# EUROPEAN PATENT SPECIFICATION

(11) **EP 0 904 380 B1**
(45) Date of publication and mention of the grant of the patent: **09.11.2005**
(21) Application number: 97906594.3
(22) Date of filing: 18.02.1997
(51) Int. Cl.: C12N 15/49, A61K 31/70, A61K 48/00, C12N 15/67

(54) **SYNTHETIC HIV GENES**
SYNTHETISCHE HIV-GENE
GENES SYNTHETIQUES DU VIH

(30) Priority: 22.02.1996 US 12082 P; 09.04.1996 GB 9607293
(43) Date of publication of application: 31.03.1999
(73) Proprietor: Merck & Co., Inc., Rahway, New Jersey 07065 (US)
(72) Inventor: SHIVER, John, W., Rahway, NJ 07065 (US); DAVIES, Mary-Ellen, Rahway, NJ 07065 (US); FREED, Daniel, C., Rahway, NJ 07065 (US); LIU, Margaret, A., Rahway, NJ 07065 (US); PERRY, Helen, C., Rahway, NJ 07065 (US)
(74) Representative: Horgan, James Michael Frederic
(86) International application number: PCT/US1997/002294
(87) International publication number: WO 1997/031115

(56) References cited:
- WO-A-94/16737
- WO-A-95/20660
- WO-A-96/21356
- K. OKUDA ET AL.: "Induction of potent humoral and cell-mediated immune responses following direct injection of DNA encoding the HIV typr 1 env and rev gene products" AIDS RESEARCH AND HUMAN RETROVIRUSES, vol. 11, no. 8, August 1995 (1995-08), pages 933-943, XP002036839
- B. WANG ET AL.: "Gene inoculation generates immune responses against human immunodeficiency virus type 1" PROCEEDINGS OF THE NATIONAL ACADEMY OF SCIENCES OF USA, vol. 90, 1 May 1993 (1993-05-01), pages 4156-4160, XP000608482 WASHINGTON US
- DATABASE WPI Section Ch, Week 9641 Derwent Publications Ltd., London, GB; Class B04, AN 96-408330 XP002036840 & JP 08 198774 A (OKUDA K), 6 August 1996 (1996-08-06)
- HASS ET AL: 'Codon usage limitation in the expression of HIV-1 envelope glycoprotein' CURRENT BIOLOGY vol. 6, no. 3, 1996, pages 315 - 324

## Description

### CROSS-REFERENCE TO RELATED APPLICATIONS

This is a nonprovisional application related to U.S. Serial No. 60/012,082, filed February 22, 1996.

### FIELD OF THE INVENTION

HIV Vaccines.

### BACKGROUND OF THE INVENTION

### 1. HIV Infection:

Human Immunodeficiency Virus-1 (HIV-1) is the etiological agent of acquired human immune deficiency syndrome (AIDS) and related disorders. HIV-1 is an RNA virus of the Retroviridae family and exhibits the 5'LTR-*gag*-pol-*env*-LTR3' organization of all retroviruses. In addition, HIV-1 comprises a handful of genes with regulatory or unknown functions, including the tat and *rev* genes. The *env* gene encodes the viral envelope glycoprotein that is translated as a 160-kilodalton (kDa) precursor (gp160) and then cleaved by a cellular protease to yield the external 120-kDa envelope glycoprotein (gp120) and the transmembrane 41-kDa envelope glycoprotein (gp41). Gp120 and gp41 remain associated and are displayed on the viral particles and the surface of HIV-infected cells. Gp120 binds to the CD4 receptor present on the surface of helper T-lymphocytes, macrophages and other target cells. After gp120 binds to CD4, gp41 mediates the fusion event responsible for virus entry.

Infection begins when gp120 on the viral particle binds to the CD4 receptor on the surface of T4 lymphocytes or other target cells. The bound virus merges with the target cell and reverse transcribes its RNA genome into the double-stranded DNA of the cell. The viral DNA is incorporated into the genetic material in the cell's nucleus, where the viral DNA directs the production of new viral RNA, viral proteins, and new virus particles. The new particles bud from the target cell membrane and infect other cells.

Destruction of T4 lymphocytes, which are critical to immune defense, is a major cause of the progressive immune dysfunction that is the hallmark of HIV infection. The loss of target cells seriously impairs the body's ability to fight most invaders, but it has a particularly severe impact on the defenses against viruses, fungi, parasites and certain bacteria, including mycobacteria.

HIV-1 kills the cells it infects by replicating, budding from them and damaging the cell membrane. HIV-1 may kill target cells indirectly by means of the viral gp120 that is displayed on an infected cell's surface. Since the CD4 receptor on T cells has a strong affinity for gp120, healthy cells expressing CD4 receptor can bind to gp120 and fuse with infected cells to form a syncytium. A syncytium cannot survive.

HIV-1 can also elicit normal cellular immune defenses against infected cells. With or without the help of antibodies, cytotoxic defensive cells can destroy an infected cell that displays viral proteins on its surface. Finally, free gp120 may circulate in the blood of individuals infected with HIV-1. The free protein may bind to the CD4 receptor of uninfected cells, making them appear to be infected and evoking an immune response.

Infection with HIV-1 is almost always fatal, and at present there are no cures for HIV-1 infection. Effective vaccines for prevention of HIV-1 infection are not yet available. Because of the danger of reversion or infection, live attenuated virus probably cannot be used as a vaccine. Most subunit vaccine approaches have not been successful at preventing HIV infection. Treatments for HIV-1 infection, while prolonging the lives of some infected persons, have serious side effects. There is thus a great need for effective treatments and vaccines to combat this lethal infection.

### 2. Vaccines

Vaccination is an effective form of disease prevention and has proven successful against several types of viral infection. Determining ways to present HIV-1 antigens to the human immune system in order to evoke protective humoral and cellular immunity, is a difficult task. To date, attempts to generate an effective HIV vaccine have not been successful. In AIDS patients, free virus is present in low levels only. Transmission of HIV-1 is enhanced by cell-to-cell interaction via fusion and syncytia formation. Hence, antibodies generated against free virus or viral subunits are generally ineffective in eliminating virus-infected cells.

Vaccines exploit the body's ability to "remember" an antigen. After first encounters with a given antigen the immune system generates cells that retain an immunological memory of the antigen for an individual's lifetime. Subsequent exposure to the antigen stimulates the immune response and results in elimination or inactivation of the pathogen.

The immune system deals with pathogens in two ways: by humoral and by cell-mediated responses. In the humoral response lymphocytes generate specific antibodies that bind to the antigen thus inactivating the pathogen. The cell-mediated response involves cytotoxic and helper T lymphocytes that specifically attack and destroy infected cells.

Vaccine development with HIV-1 virus presents problems because HIV-1 infects some of the same cells the vaccine needs to activate in the immune system (i.e., T4 lymphocytes). It would be advantageous to develop a vaccine which inactivates HIV before impairment of the immune system occurs. A particularly suitable type of HIV vaccine would generate an anti-HIV immune response which recognizes HIV variants and which works in HIV-positive individuals who are at the beginning of their infection.

A major challenge to the development of vaccines against viruses, particularly those with a high rate of mutation such as the human immunodeficiency virus, against which elicitation of neutralizing and protective immune responses is desirable, is the diversity of the viral envelope proteins among different viral isolates or strains. Because cytotoxic T-lymphocytes (CTLs) in both mice and humans are capable of recognizing epitopes derived from conserved internal viral proteins, and are thought to be important in the immune response against viruses, efforts have been directed towards the development of CTL vaccines capable of providing heterologous protection against different viral strains.

It is known that CD8⁺ CTLs kill virally-infected cells when their T cell receptors recognize viral peptides associated with MHC class I molecules. The viral peptides are derived from endogenously synthesized viral proteins, regardless of the protein's location or function within the virus. Thus, by recognition of epitopes from conserved viral proteins, CTLs may provide cross-strain protection. Peptides capable of associating with MHC class I for CTL recognition originate from proteins that are present in or pass through the cytoplasm or endoplasmic reticulum. In general, exogenous proteins, which enter the endosomal processing pathway (as in the case of antigens presented by MHC class II molecules), are not effective at generating CD8⁺ CTL responses.

Most efforts to generate CTL responses have used replicating vectors to produce the protein antigen within the cell or they have focused upon the introduction of peptides into the cytosol. These approaches have limitations that may reduce their utility as vaccines. Retroviral vectors have restrictions on the size and structure of polypeptides that can be expressed as fusion proteins while maintaining the ability of the recombinant virus to replicate, and the effectiveness of vectors such as vaccinia for subsequent immunizations may be compromised by immune responses against the vectors themselves. Also, viral vectors and modified pathogens have inherent risks that may hinder their use in humans. Furthermore, the selection of peptide epitopes to be presented is dependent upon the structure of an individual's MHC antigens and, therefore, peptide vaccines may have limited effectiveness due to the diversity of MHC haplotypes in outbred populations.

### 3. DNA Vaccines

Benvenisty, N., and Reshef, L. [PNAS **83,** 9551-9555, (1986)] showed that CaCl₂-precipitated DNA introduced into mice intraperitoneally (i.p.), intravenously (i.v.) or intramuscularly (i.m.) could be expressed. The i.m. injection of DNA expression vectors without CaCl₂ treatment in mice resulted in the uptake of DNA by the muscle cells and expression of the protein encoded by the DNA . The plasmids were maintained episomally and did not replicate. Subsequently, persistent expression has been observed after i.m. injection in skeletal muscle of rats, fish and primates, and cardiac muscle of rats. The technique of using nucleic acids as therapeutic agents was reported in WO90/11092 (4 October 1990), in which naked polynucleotides were used to vaccinate vertebrates.

It is not necessary for the success of the method that immunization be intramuscular. The introduction of gold microprojectiles coated with DNA encoding bovine growth hormone (BGH) into the skin of mice resulted in production of anti-BGH antibodies in the mice. A jet injector has been used to transfect skin, muscle, fat, and mammary tissues of living animals. Various methods for introducing nucleic have been reviewed. Intravenous injection of a DNA:cationic liposome complex in mice was shown by Zhu et al., [Science 261:209-211 (9 July 1993) to result in systemic expression of a cloned transgene. Ulmer et al., [Science 259:1745-1749, (1993)] reported on the heterologous protection against influenza virus infection by intramuscular injection of DNA encoding influenza virus proteins.

The need for specific therapeutic and prophylactic agents capable of eliciting desired immune responses against pathogens and tumor antigens is met by the instant invention. Of particular importance in this therapeutic approach is the ability to induce T-cell immune responses which can prevent infections or disease caused even by virus strains which are heterologous to the strain from which the antigen gene was obtained. This is of particular concern when dealing with HIV as this virus has been recognized to mutate rapidly and many virulent isolates have been identified [see, for example, LaRosa et al., Science 249:932-935 (1990), identifying 245 separate HIV isolates]. In response to this recognized diversity, researchers have attempted to generate CTLs based on peptide immunization. Thus, Takahashi et al., [Science 255 :333-336 (1992)] reported on the induction of broadly cross-reactive cytotoxic T cells recognizing an HIV envelope (gp160) determinant. However, those workers recognized the difficulty in achieving a truly cross-reactive CTL response and suggested that there is a dichotomy between the priming or restimulation of T cells, which is very stringent, and the elicitation of effector function, including cytotoxicity, from already stimulated CTLs.

Wang et al. reported on elicitation of immune responses in mice against HIV by intramuscular inoculation with a cloned, genomic (unspliced) HIV gene. However, the level of immune responses achieved in these studies was very low. In addition, the Wang et al., DNA construct utilized an essentially genomic piece of HIV encoding contiguous Tat/*REV*-gp160-Tat/*REV* coding sequences. As is described in detail below, this is a suboptimal system for obtaining high-level expression of the gp160. It also is potentially dangerous because expression of Tat contributes to the progression of Kaposi's Sarcoma.

WO 93/17706 describes a method for vaccinating an animal against a virus, wherein carrier particles were coated with a gene construct and the coated particles are accelerated into cells of an animal. In regard to HIV, essentially the entire genome, minus the long terminal repeats, was proposed to be used. That method represents substantial risks for recipients. It is generally believed that constructs of HIV should contain less than about 50% of the HIV genome to ensure safety of the vaccine; this ensures that enzymatic moieties and viral regulatory proteins, many of which have unknown or poorly understood functions have been eliminated. Thus, a number of problems remain if a useful human HIV vaccine is to emerge from the gene-delivery technology.

The instant invention contemplates any of the known methods for introducing polynucleotides into living tissue to induce expression of proteins. However, this invention provides a novel immunogen for introducing HIV and other proteins into the antigen processing pathway to efficiently generate HIV-specific CTLs and antibodies. The pharmaceutical is effective as a vaccine to induce both cellular and humoral anti-HIV and HIV neutralizing immune responses. In the instant invention, the problems noted above are addressed and solved by the provision of polynucleotide immunogens which, when introduced into an animal, direct the efficient expression of HIV proteins and epitopes without the attendant risks associated with those methods. The immune responses thus generated are effective at recognizing HIV, at inhibiting replication of HIV, at identifying and killing cells infected with HIV, and are cross-reactive against many HIV strains.

### 4. Codon Usage and Codon Context

The codon pairings of organisms are highly nonrandom, and differ from organism to organism. This information is used to construct and express altered or synthetic genes having desired levels of translational efficiency, to determine which regions in a genome are protein coding regions, to introduce translational pause sites into heterologous genes, and to ascertain relationship or ancestral origin of nucleotide sequences

The expression of foreign heterologous genes in transformed organisms is now commonplace. A large number of mammalian genes, including, for example, murine and human genes, have been successfully inserted into single celled organisms. Standard techniques in this regard include introduction of the foreign gene to be expressed into a vector such as a plasmid or a phage and utilizing that vector to insert the gene into an organism. The native promoters for such genes are commonly replaced with strong promoters compatible with the host into which the gene is inserted. Protein sequencing machinery permits elucidation of the amino acid sequences of even minute quantities of native protein. From these amino acid sequences, DNA sequences coding for those proteins can be inferred. DNA synthesis is also a rapidly developing art, and synthetic genes corresponding to those inferred DNA sequences can be readily constructed.

Despite the burgeoning knowledge of expression systems and recombinant DNA, significant obstacles remain when one attempts to express a foreign or synthetic gene in an organism. Many native, active proteins, for example, are glycosylated in a manner different from that which occurs when they are expressed in a foreign host. For this reason, eukaryotic hosts such as yeast may be preferred to bacterial hosts for expressing many mammalian genes. The glycosylation problem is the subject of continuing research.

Another problem is more poorly understood. Often translation of a synthetic gene, even when coupled with a strong promoter, proceeds much less efficiently than would be expected. The same is frequently true of exogenous genes foreign to the expression organism. Even when the gene is transcribed in a sufficiently efficient manner that recoverable quantities of the translation product are produced, the protein is often inactive or otherwise different in properties from the native protein.

It is recognized that the latter problem is commonly due to differences in protein folding in various organisms. The solution to this problem has been elusive, and the mechanisms controlling protein folding are poorly understood.

The problems related to translational efficiency are believed to be related to codon context effects. The protein coding regions of genes in all organisms are subject to a wide variety of functional constraints, some of which depend on the requirement for encoding a properly functioning protein, as well as appropriate translational start and stop signals. However, several features of protein coding regions have been discerned which are not readily understood in terms of these constraints. Two important classes of such features are those involving codon usage and codon context.

It is known that codon utilization is highly biased and varies considerably between different organisms. Codon usage patterns have been shown to be related to the relative abundance of tRNA isoacceptors. Genes encoding proteins of high versus low abundance show differences in their codon preferences. The possibility that biases in codon usage alter peptide elongation rates has been widely discussed. While differences in codon use are associated with differences in translation rates, direct effects of codon choice on translation have been difficult to demonstrate. Other proposed constraints on codon usage patterns include maximizing the fidelity of translation and optimizing the kinetic efficiency of protein synthesis.

Apart from the non-random use of codons, considerable evidence has accumulated that codon/anticodon recognition is influenced by sequences outside the codon itself, a phenomenon termed "codon context." There exists a strong influence of nearby nucleotides on the efficiency of suppression of nonsense codons as well as missense codons. Clearly, the abundance of suppressor activity in natural bacterial populations, as well as the use of "termination" codons to encode selenocysteine and phosphoserine require that termination be context-dependent. Similar context effects have been shown to influence the fidelity of translation, as well as the efficiency of translation initiation.

Statistical analyses of protein coding regions of E. coli have demonstrate another manifestation of "codon context." The presence of a particular codon at one position strongly influences the frequency of occurrence of certain nucleotides in neighboring codons, and these context constraints differ markedly for genes expressed at high versus low levels. Although the context effect has been recognized, the predictive value of the statistical rules relating to preferred nucleotides adjacent to codons is relatively low. This has limited the utility of such nucleotide preference data for selecting codons to effect desired levels of translational efficiency.

The advent of automated nucleotide sequencing equipment has made available large quantities of sequence data for a wide variety of organisms. Understanding those data presents substantial difficulties. For example, it is important to identify the coding regions of the genome in order to relate the genetic sequence data to protein sequences. In addition, the ancestry of the genome of certain organisms is of substantial interest. It is known that genomes of some organisms are of mixed ancestry. Some sequences that are viral in origin are now stably incorporated into the genome of eukaryotic organisms. The viral sequences themselves may have originated in another substantially unrelated species. An understanding of the ancestry of a gene can be important in drawing proper analogies between related genes and their translation products in other organisms.

There is a need for a better understanding of codon context effects on translation, and for a method for determining the appropriate codons for any desired translational effect. There is also a need for a method for identifying coding regions of the genome from nucleotide sequence data. There is also a need for a method for controlling protein folding and for insuring that a foreign gene will fold appropriately when expressed in a host. Genes altered or constructed in accordance with desired translational efficiencies would be of significant worth.

Another aspect of the practice of recombinant DNA techniques for the expression by microorganisms of proteins of industrial and pharmaceutical interest is the phenomenon of "codon preference". While it was earlier noted that the existing machinery for gene expression is genetically transformed host cells will "operate" to construct a given desired product, levels of expression attained in a microorganism can be subject to wide variation, depending in part on specific alternative forms of the amino acid-specifying genetic code present in an inserted exogenous gene. A "triplet" codon of four possible nucleotide bases can exist in 64 variant forms. That these forms provide the message for only 20 different amino acids (as well as transcription initiation and termination) means that some amino acids can be coded for by more than one codon. Indeed, some amino acids have as many as six "redundant", alternative codons while some others have a single, required codon. For reasons not completely understood, alternative codons are not at all uniformly present in the endogenous DNA of differing types of cells and there appears to exist avariable natural hierarchy or "preference" for certain codons in certain types of cells.

As one example, the amino acid leucine is specified by any of six DNA codons including CTA, CTC, CTG, CTT, TTA, and TTG (which correspond, respectively, to the mRNA codons, CUA, CUC, CUG, CUU, UUA and UUG). Exhaustive analysis of genome codon frequencies for microorganisms has revealed endogenous DNA of E. coli most commonly contains the CTG leucine-specifying codon, while the DNA of yeasts and slime molds most commonly includes a TTA leucine-specifying codon. In view of this hierarchy, it is generally held that the likelihood of obtaining high levels of expression of a leucine-rich polypeptide by an E. coli host will depend to some extent on the frequency of codon use. For example, a gene rich in TTA codons will in all probability be poorly expressed in E. coli, whereas a CTG rich gene will probably highly express the polypeptide. Similarly, when yeast cells are the projected transformation host cells for expression of a leucine-rich polypeptide, a preferred codon for use in an inserted DNA would be TTA.

The implications of codon preference phenomena on recombinant DNA techniques are manifest, and the phenomenon may serve to explain many prior failures to achieve high expression levels of exogenous genes in successfully transformed host organisms-a less "preferred" codon may be repeatedly present in the inserted gene and the host cell machinery for expression may not operate as efficiently. This phenomenon suggests that synthetic genes which have been designed to include a projected host cell's preferred codons provide a preferred form of foreign genetic material for practice of recombinant DNA techniques.

### 5. Protein Trafficking

The diversity of function that typifies eukarycate cells depends upon the structural differentiation of their membrane boundaries. To generate and maintain these structures, proteins must be transported from their site of synthesis in the endoplasmic reticulum to predetermined destinations throughout the cell. This requires that the trafficking proteins display sorting signals that are recognized by the molecular machinery responsible for route selection located at the access points to the main trafficking pathways. Sorting decisions for most proteins need to be made only once ase they traverse their biosynthetic pathways since their final destination, the cellular location at which they perform their function, becomes their permanent residence.

Maintenance of intracellular integrity depends in part on the selective sorting and accurate transport of proteins to their correct destinations. Over the past few years the dissection of the molecular machinery for targeting and localization of proteins has been studied vigorously. Defined sequence motifs have been identified on proteins which can act as 'address labels'. A number of sorting signals have been found associated with the cytoplasmic domains of membrane proteins.

### SUMMARY OF THE INVENTION

Synthetic DNA molecules encoding HIV *env* and modifications of HIV *env* are provided. The codons of the synthetic molecules include the projected host cell's preferred codons. The synthetic molecules provide preferred forms of foreign genetic material. The synthetic molecules may be used as a polynucleotide vaccine which provides effective immunoprophylaxis against HIV infection through neutralizing antibody and cell-mediated immunity. This invention provides polynucleotides which, when directly introduced into a vertebrate in vivo, including mammals such as primates and humans, induce the expression of encoded proteins within the animal.

Specifically, there is provided a synthetic polynucleotide as defined claim 1.

### BRIEF DESCRIPTION OF THE DRAWINGS

Figure 1 shows HIV *env* cassette-based expression strategies.

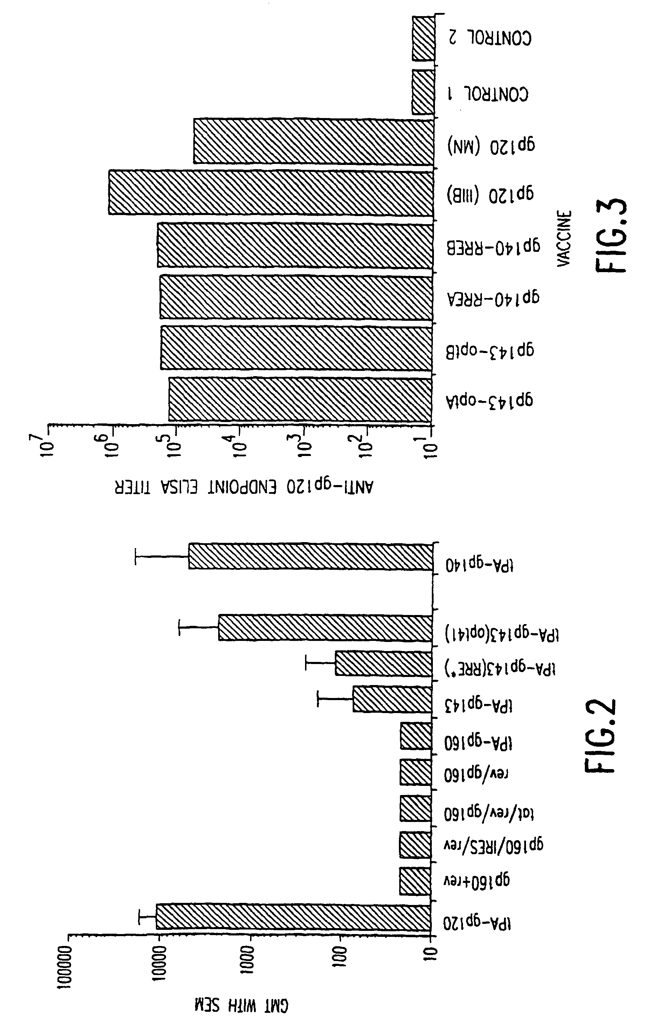

Figure 2 shows DNA vaccine mediated anti-gp120 responses.
Figure 3 shows anti-gp120 ELISA titers of murine DNA vaccinee sera.

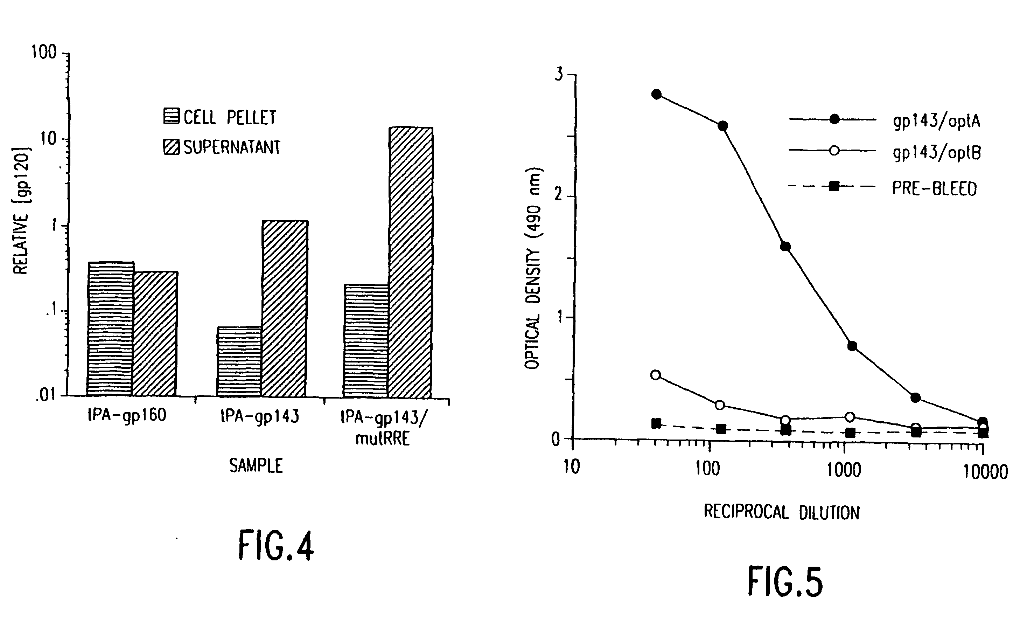

Figure 4 shows the relative expression of gp120 after HIV *env* PNV cell culture transfection.
Figure 5 shows the mean anti-gp120 ELISA responses following tPA-gp143/optA vs. optB DNA vaccination.

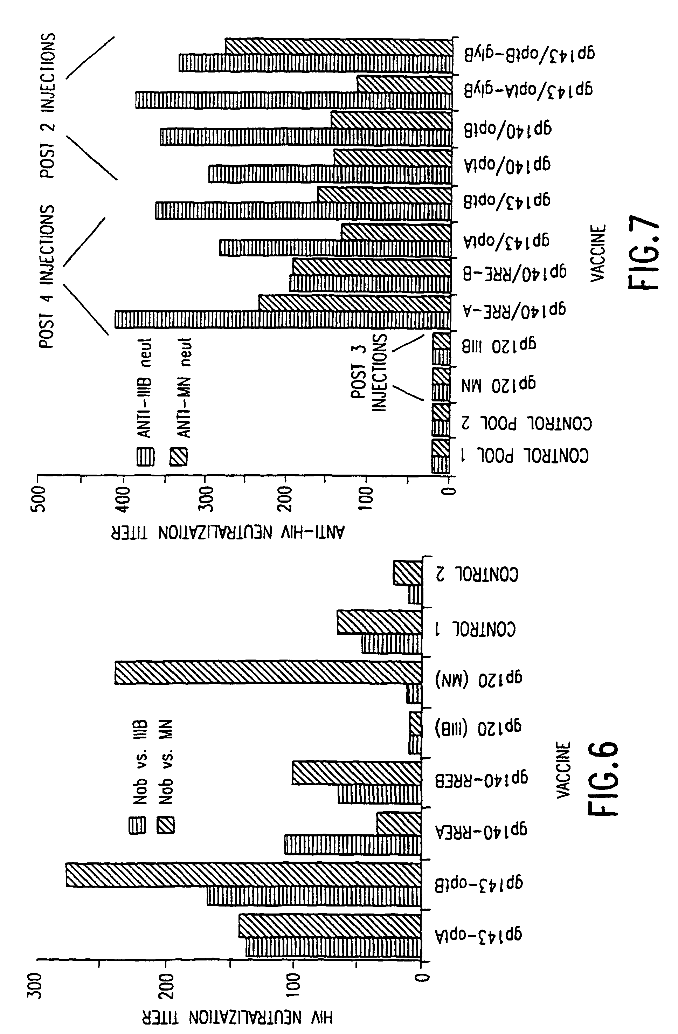

Figure 6 shows the neutralization of HIV by murine DNA vaccinee sera.
Figure 7 shows HIV neutralization by sera from murine HIV *env* DNA vaccinees.

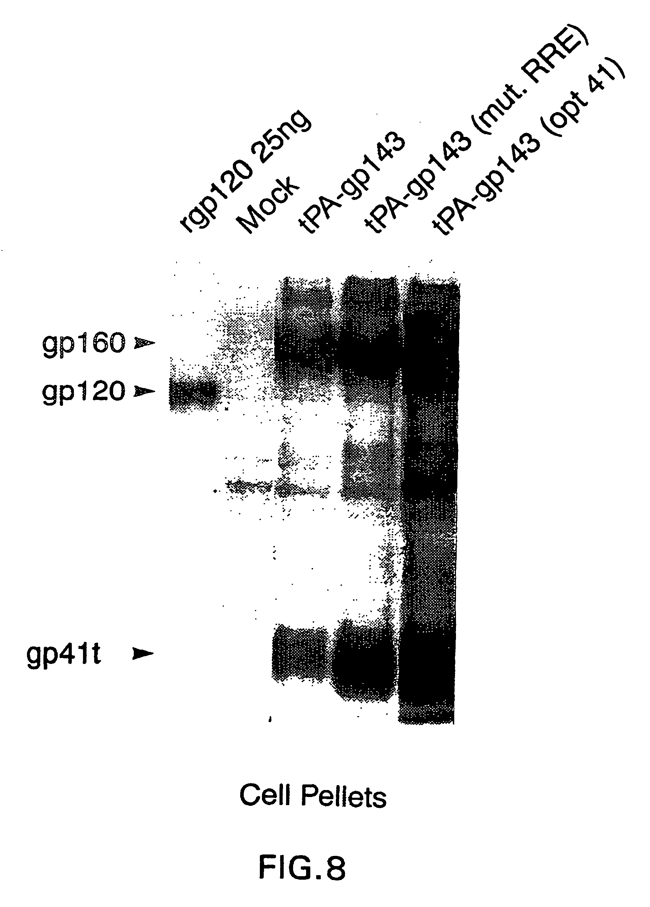

Figure 8 is an immunoblot analysis of optimized HIV *env* DNA constructs.

### DETAILED DESCRIPTION OF THE INVENTION

Synthetic DNA molecules encoding HIV *env* and synthetic DNA molecules encoding modified forms of HIV *env* are provided. The codons of the synthetic molecules are designed so as to use the codons preferred by the projected host cell. The synthetic molecules may be used as a polynucleotide vaccine which provides effective immunoprophylaxis against HIV infection through neutralizing antibody and cell-mediated immunity. The synthetic molecules may be used as an immunogenic composition. This invention provides polynucleotides which, when directly introduced into a vertebrate in vivo, including mammals such as primates and humans, induce the expression of encoded proteins within the animal.

As used herein, a polynucleotide is a combination of nucleic acids which contain regulatory elements such that upon introduction into a living, vertebrate cell, the polynucleotide is able to direct cellular machinery to produce translation products encoded by the nucleic acids comprising the polynucleotide. In one embodiment of the invention, the polynucleotide is a polydeoxyribonucleic acid comprising at least one HIV gene operatively linked to a transcriptional promoter. In another embodiment of the invention, the polynucleotide vaccine (PNV) comprises polyribonucleic acid encoding at least one HIV gene which is amenable to translation by the eukaryotic cellular machinery (ribosomes, tRNAs, and other translation factors). Where the protein encoded by the polynucleotide is one which does not normally occur in that animal except in pathological conditions, (i.e., a heterologous protein) such as proteins associated with human immunodeficiency virus, (HIV), the etiologic agent of acquired immune deficiency syndrome, (AIDS), the animals' immune system is activated to launch a protective immune response. Because these exogenous proteins are produced by the animals' tissues, the expressed proteins are processed by the major histocompatibility system, MHC, in a fashion analogous to when an actual infection with the related organism (HIV) occurs. The result is induction of immune responses against the cognate pathogen.

The polynucleotides of the instant disclosure, when introduced into a biological system, induce the expression of HIV proteins and epitopes and the production of a specific immune response. The induced antibody response is specific for the expressed HIV protein and neutralizes HIV. In addition, cytotoxic T-lymphocytes (CTL) which specifically recognize and destroy HIV infected cells are induced.

The instant disclosure provides methods for using a polynucleotide which, upon introduction into mammalian tissue, induces the expression in a single cell, in vivo, of discrete gene products. The disclosure provides a solution which does not require multiple manipulations of *REV* dependent HIV genes to obtain *REV*-independent genes. The *REV*-independent expression system described herein is useful in its own right and is a system for demonstrating the expression in a single cell in vivo of a single desired gene-product.

Because many of the applications of the instant invention apply to antiviral vaccination, the polynucleotides are frequently referred to as polynucleotide vaccine(s), or PNV. This is not to say that additional utilities of these polynucleotides, in immune stimulation and in antitumor therapeutics, are considered to be outside the scope of the invention.

In accordance with the invention, a gene encoding an HIV gene product is incorporated in an expression vector. The vector contains a transcriptional promoter recognized by an eukaryotic RNA polymerase, and a transcriptional terminator at the end of the HIV gene coding sequence. In a preferred embodiment, the promoter is the cytomegalovirus promoter with the intron A sequence (CMV-intA), although those skilled in the art will recognize that any of a number of other known promoters such as the strong immunoglobulin, or other eukaryotic gene promoters may be used. A preferred transcriptional terminator is the bovine growth hormone terminator. The combination of CMVintA-BGH terminator is particularly preferred.

To assist in preparation of the polynucleotides in prokaryotic cells, an antibiotic resistance marker may be included in the expression vector; the marker may be under transcriptional control of a prokaryotic promoter so that expression of the marker does not occur in eukaryotic cells. Ampicillin-resistance genes, neomycin-resistance genes and other pharmaceutically acceptable antibiotic resistance markers may be used. To aid in the production of high levels of the polynucleotide by fermentation in prokaryotic organisms, it may be advantageous for the vector to contain a prokaryotic origin of replication and be of high copy number. A number of commercially-available prokaryotic cloning vectors provide these benefits. It is desirable to remove non-essential DNA sequences and to ensure that the vectors are not able to replicate in eukaryotic cells. This minimizes the risk of integration of polynucleotide vaccine sequences into the recipients' genome. Tissue-specific promoters or enhancers may be used whenever it is desirable to limit expression of the polynucleotide to a particular tissue type.

Examples of vectors include V1, a mutated pBR322 vector into which the CMV promoter and the BGH transcriptional terminator were cloned. In another vector, the elements of V1 and pUC19 have been been combined to produce an expression vector named V1J. In another refinement, the ampicillin resistance gene is removed from V1J and replaced with a neomycin resistance gene, to generate V1J-neo. In the invention, the vector is V1Jns, which is the same as V1Jneo except that a unique Sfi1 restriction site has been engineered into the single Kpn 1 site at position 2114 of V 1J-neo. The incidence of Sfi 1 sites in human genomic DNA is very low (aproximately 1 site per 100,000 bases). Thus, this vector allows careful monitoring for expression vector integration into host DNA, simply by Sfi1 digestion of extracted genomic DNA. In a further refinement, the vector is V1R. In this vector, as much non-essential DNA as possible was "trimmed" from the vector to produce a highly compact vector. This vector is a derivative of V1Jns. This vector allows larger inserts to be used, with less concern that undesirable sequences are encoded and optimizes uptake by cells.

One embodiment of this invention incorporates genes encoding HIV gp160, gp120, *gag* and other gene products from laboratory adapted strains of HIV such as SF2, IIIB or MN. Those skilled in the art will recognize that the use of genes from HIV-2 strains having analogous function to the genes from HIV-1 would be expected to generate immune responses analagous to those described herein for HIV-1 constructs. The cloning and manipulation methods for obtaining these genes are known to those skilled in the art.

It is recognized that elicitation of immune responses against laboratory adapted strains of HIV may not be adequate to provide neutralization of primary field isolates of HIV. Thus, in another embodiment of this invention, genes from virulent, primary field isolates of HIV are incorporated in the polynucleotide immunogen. This is accomplished by preparing cDNA copies of the viral genes and then subcloning the individual genes into the polynucleotide immunogen. Sequences for many genes of many HIV strains are now publicly available on GENBANK and such primary, field isolates of HIV are available from the National Institute of Allergy and Infectious Diseases (NIAID) which has contracted with Quality Biological, Inc., [7581 Lindbergh Drive, Gaithersburg, Maryland 20879] to make these strains available. Such strains are also available from the World Health Organization (WHO) [Network for HIV Isolation and Characterization, Vaccine Development Unit, Office of Research, Global Programme on AIDS, CH-1211 Geneva 27, Switzerland]. From this work those skilled in the art will recognize that one of the utilities of the instant invention is to provide a system for in vivo as well as in vitro testing and analysis so that a correlation of HIV sequence diversity with serology of HIV neutralization, as well as other parameters can be made. Incorporation of genes from primary isolates of HIV strains provides an immunogen which induces immune responses against clinical isolates of the virus and thus meets a need as yet unmet in the field. Furthermore, as the virulent isolates change, the immunogen may be modified to reflect new sequences as necessary.

To keep the terminology consistent, the following convention is followed herein for describing polynucleotide immunogen constructs: "Vector name-HIV strain-gene-additional elements". Thus, a construct wherein the gp160 gene of the MN strain is cloned into the expression vector V1Jneo, the name it is given herein is: "V1Jneo-MN-gp160". The additional elements that are added to the construct are described in further detail below. As the etiologic strain of the virus changes, the precise gene which is optimal for incorporation in the pharmaceutical may be changed. However, as is demonstrated below, because CTL responses are induced which are capable of protecting against heterologous strains, the strain variability is less critical in the immunogen and vaccines of this invention, as compared with the whole virus or subunit polypeptide based vaccines. In addition, because the pharmaceutical is easily manipulated to insert a new gene, this is an adjustment which is easily made by the standard techniques of molecular biology.

The term "promoter" as used herein refers to a recognition site on a DNA strand to which the RNA polymerase binds. The promoter forms an initiation complex with RNA polymerase to initiate and drive transcriptional activity. The complex can be modified by activating sequences termed "enhancers" or inhibiting sequences termed "silencers."

The term "leader" as used herein refers to a DNA sequence at the 5' end of a structural gene which is transcribed along with the gene. The leader usually results in the protein having an N-terminal peptide extension sometimes called a pro-sequence. For proteins destined for either secretion to the extracellular medium or a membrane, this signal sequence, which is generally hydrophobic, directs the protein into endoplasmic reticulum from which it is discharged to the appropriate destination.

The term "intron" as used herein refers to a section of DNA occurring in the middle of a gene which does not code for an amino acid in the gene product. The precursor RNA of the intron is excised and is therefore not transcribed into mRNA nor translated into protein.

The term "cassette" refers to the sequence of the present invention which contains the nucleic acid sequence which is to be expressed. The cassette is similar in concept to a cassette tape. Each cassette will have its own sequence. Thus by interchanging the cassette the vector will express a different sequence. Because of the restrictions sites at the 5' and 3' ends, the cassette can be easily inserted, removed or replaced with another cassette.

The term "3' untranslated region" or "3' UTR" refers to the sequence at the 3' end of a structural gene which is usually transcribed with the gene. This 3' UTR region usually contains the poly A sequence. Although the 3' UTR is transcribed from the DNA it is excised before translation into the protein.

The term "Non-Coding Region" or "NCR" refers to the region which is contiguous to the 3' UTR region of the structural gene. The NCR region contains a transcriptional termination signal.

The term "restriction site" refers to a sequence specific cleavage site of restriction endonucleases.

The term "vector" refers to some means by which DNA fragments can be introduced into a host organism or host tissue. There are various types of vectors including plasmid, bacteriophages and cosmids.

The term "effective amount" means sufficient PNV is injected to produce the adequate levels of the polypeptide. One skilled in the art recognizes that this level may vary.

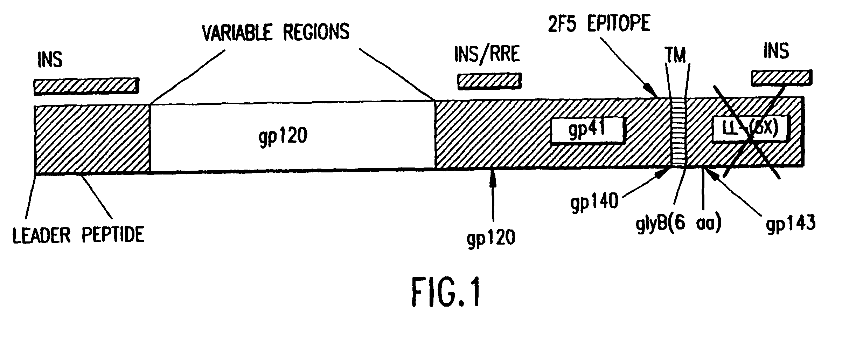

To provide a description of the instant invention, the following background on HIV is provided. The human immunodeficiency virus has a ribonucleic acid (RNA) genome, the structure of which is represented in Figure 1. This RNA genome must be reverse transcribed according to methods known in the art in order to produce a cDNA copy for cloning and manipulation according to the methods taught herein. At each end of the genome is a long terminal repeat which acts as a promoter. Between these termini, the genome encodes, in various reading frames, ***gag*****-pol-*****env*** as the major gene products: *gag* is the group specific antigen; pol is the reverse transcriptase, or polymerase; also encoded by this region, in an alternate reading frame, is the viral protease which is responsible for post-translational processing, for example, of gp160 into gp120 and gp41; *env* is the envelope protein; vif is the virion infectivity factor; *REV* is the regulator of virion protein expression; neg is the negative regulatory factor; vpu is the virion productivity factor "u"; tat is the trans-activator of transcription; vpr is the viral protein r. The function of each of these elements has been described.

In one embodiment of this invention, a gene encoding an HIV or SIV protein is directly linked to a transcriptional promoter. The *env* gene encodes a large, membrane bound protein, gp160, which is post-translationally modified to gp41 and gp120. The gp120 gene may be placed under the control of the cytomegalovirus promoter for expression. However, gp120 is not membrane bound and therefore, upon expression, it may be secreted from the cell. As HIV tends to remain dormant in infected cells, it is desirable that immune responses directed at cell-bound HIV epitopes also be generated. Additionally, it is desireable that a vaccine produce membrane bound, oligomeric *ENV* antigen similar in structure to that produced by viral infection in order to generate the most efficacious antibody responses for viral neutralization. This goal is accomplished herein by expression in vivo of the cell-membrane associated epitope, gp160, to prime the immune system. However, expression of gp160 is repressed in the absence of *REV* due to non-export from the nucleus of non-spliced genes. For an understanding of this system, the life cycle of HIV must be described in further detail.

In the life cycle of HIV, upon infection of a host cell, HIV RNA genome is reverse-transcribed into a proviral DNA which integrates into host genomic DNA as a single transcriptional unit. The LTR provides the promoter which transcribes HIV genes from the 5' to 3' direction (*gag*, pol, *env*), to form an unspliced transcript of the entire genome. The unspliced transcript functions as the mRNA from which *gag* and pol are translated, while limited splicing must occur for translation of *env* encoded genes. For the regulatory gene product *REV* to be expressed, more than one splicing event must occur because in the genomic setting, *REV* and *env,* as is shown in figure 1, overlap. In order for transcription of *env* to occur, *REV* transcription must stop, and vice versa. In addition, the presence of *REV* is required for export of unspliced RNA from the nucleus. For *REV* to function in this manner, however, a *REV* responsive element (RRE) must be present on the transcript [Malim et al., Nature 338:254-257 (1989)].

In the polynucleotide vaccine of this invention, the obligatory splicing of certain HIV genes is eliminated by providing fully spliced genes (i.e.: the provision of a complete open reading frame for the desired gene product without the need for switches in the reading frame or elimination of noncoding regions; those of ordinary skill in the art would recognize that when splicing a particular gene, there is some latitude in the precise sequence that results; however so long as a functional coding sequence is obtained, this is acceptable). Thus, in one embodiment, the entire coding sequence for gp160 is spliced such that no intermittent expression of each gene product is required.

The dual humoral and cellular immune responses generated according to this invention are particularly significant to inhibiting HIV infection, given the propensity of HIV to mutate within the population, as well as in infected individuals. In order to formulate an effective protective vaccine for HIV it is desirable to generate both a multivalent antibody response for example to gp160 (*env* is approximately 80% conserved across various HIV-1, clade B strains, which are the prevalent strains in US human populations), the principal neutralization target on HIV, as well as cytotoxic T cells reactive to the conserved portions of gp160 and, internal viral proteins encoded by *gag*. We have made an HIV vaccine comprising gp160 genes selected from common laboratory strains; from predominant, primary viral isolates found within the infected population; from mutated gp160s designed to unmask cross-strain, neutralizing antibody epitopes; and from other representative HIV genes such as the *gag* and *pol* genes (~95% conserved across HIV isolates.

Virtually all HIV seropositive patients who have not advanced towards an immunodeficient state harbor anti-*gag* CTLs while about 60% of these patients show cross-strain, gp160-specific CTLs. The amount of HIV specific CTLs found in infected individuals that have progressed on to the disease state known as AIDS, however, is much lower, demonstrating the significance of our findings that we can induce cross-strain CTL responses.

Immune responses induced by our *env* and*gag* polynucleotide vaccine constructs are demonstrated in mice and primates. Monitoring antibody production to *env* in mice allows confirmation that a given construct is suitably immunogenic, i.e., a high proportion of vaccinated animals show an antibody response. Mice also provide the most facile animal model suitable for testing CTL induction by our constructs and are therefore used to evaluate whether a particular construct is able to generate such activity. Monkeys (African green, rhesus, chimpanzees) provide additional species including primates for antibody evaluation in larger, non-rodent animals. These species are also preferred to mice for antisera neutralization assays due to high levels of endogenous neutralizing activities against retroviruses observed in mouse sera. These data demonstrate that sufficient immunogenicity is engendered by our vaccines to achieve protection in experiments in a chimpanzee/HIV_{IIIB} challenge model based upon known protective levels of neutralizing antibodies for this system. However, the currently emerging and increasingly accepted definition of protection in the scientific community is moving away from so-called "sterilizing immunity", which indicates complete protection from HIV infection, to prevention of disease. A number of correlates of this goal include reduced blood viral titer, as measured either by HIV reverse transcriptase activity, by infectivity of samples of serum, by ELISA assay of p24 or other HIV antigen concentration in blood, increased CD4⁺ T-cell concentration, and by extended survival rates [see, for example, Cohen, J., Science 262:1820-1821, 1993, for a discussion of the evolving definition of anti-HIV vaccine efficacy]. The immunogens of the instant invention also generate neutralizing immune responses against infectious (clinical, primary field) isolates of HIV.

### Immunology

### A. Antibody Responses to env.

1. gp160 and gp120. An ELISA assay is used to determine whether vaccine vectors expressing either secreted gp120 or membrane-bound gp160 are efficacious for production of *env*-specific antibodies. Initial in vitro characterization of *env* expression by our vaccination vectors is provided bu immunoblot analysis of gp160 transfected cell lysates. These data confirm and quantitate gp160 expression using anti-gp41 and anti-gp120 monoclonal antibodies to visualize transfectant cell gp160 expression. In one embodiment of this invention, gp160 is preferred to gp120 for the following reasons: (1) an initial gp120 vector gave inconsistent immunogenicity in mice and was very poorly or non-responsive in African green Monkeys; (2) gp160 contributes additional neutralizing antibody as well as CTL epitopes by providing the addition of approximately 190 amino acid residues due to the inclusion of gp41; (3) gp160 expression is more similar to viral *env* with respect to tetramer assembly and overall conformation, which may provide oligomer-dependent neutralization epitopes; and (4) we find that, like the success of membrane-bound, influenza HA constructs for producing neutralizing antibody responses in mice, ferrets, and nonhuman primates anti-gp160 antibody generation is superior to anti-gp 120 antibody generation. Selection of which type of *env* , or whether a cocktail of *env* subfragments, is preferred is determined by the experiments outlined below.
2. Presence and Breadth of Neutralizing Activity. ELISA positive antisera from monkeys is tested and shown to neutralize both homologous and heterologous HIV strains.
3. V3 vs. non-V3 Neutralizing Antibodies. A major goal for *env* PNVs is to generate broadly neutralizing antibodies. It has now been shown that antibodies directed against V3 loops are very strain specific, and the serology of this response has been used to define strains.
   a. Non-V3 neutralizing antibodies appear to primarily recognize discontinuous, structural epitopes within gp120 which are responsible for CD4 binding. Antibodies to this domain are polyclonal and more broadly cross-neutralizing probably due to restraints on mutations imposed by the need for the virus to bind its cellular ligand. An in vitro assay is used to test for blocking gp120 binding to CD4 immobilized on 96 well plates by sera from immunized animals. A second in vitro assay detects direct antibody binding to synthetic peptides representing selected V3 domains immobilized on plastic. These assays are compatible for antisera from any of the animal types used in our studies and define the types of neutralizing antibodies our vaccines have generated as well as provide an in vitro correlate to virus neutralization.
   b. gp41 harbors at least one major neutralization determinant, corresponding to the highly conserved linear epitope recognized by the broadly neutralizing 2F5 monoclonal antibody (commercially available from Viral Testing Systems Corp., Texas Commerce Tower, 600 Travis Street, Suite 4750, Houston, TX 77002-3005(USA), or Waldheim Pharmazeutika GmbH, Boltzmangasse 11, A-1091 Wien, Austria), as well as other potential sites including the well-conserved "fusion peptide" domain located at the N-terminus of gp41. Besides the detection of antibodies directed against gp41 by immunoblot as described above, an in vitro assay test is used for antibodies which bind to synthetic peptides representing these domains immobilized on plastic.
4. Maturation of the Antibody Response. In HIV seropositive patients, the neutralizing antibody responses progress from chiefly anti-V3 to include more broadly neutralizing antibodies comprising the structural gp120 domain epitopes described above (#3), including gp41 epitopes. These types of antibody responses are monitored over the course of both time and subsequent vaccinations.

### B. T Cell Reactivities Against env and gag.

1. Generation of CTL Responses. Viral proteins which are synthesized within cells give rise to MHC I-restricted CTL responses. Each of these proteins elicits CTL in seropositive patients. Our vaccines also are able to elicit CTL in mice. The immunogenetics of mouse strains are conducive to such studies, as demonstrated with influenza NP. Several epitopes have been defined for the HIV proteins *env, REV, nef* and gag in Balb/c mice, thus facilitating in vitro CTL culture and cytotoxicity assays. It is advantageous to use syngeneic tumor lines, such as the murine mastocytoma P815, transfected with these genes to provide targets for CTL as well as for in vitro antigen specific restimulation. Methods for defining immunogens capable of eliciting MHC class I-restricted cytotoxic T lymphocytes are known. A peptide encompassing amino acids 152-176 was also found to induce HIV neutralizing antibodies], and these methods may be used to identify immunogenic epitopes for inclusion in the PNV of this invention. Alternatively, the entire gene encoding gp160, gp120, protease, or *gag* could be used. As used herein, T-cell effector function is associated with mature T-cell phenotype, for example, cytotoxicity, cytokine secretion for B-cell activation, and/or recruitment or stimulation of macrophages and neutrophils.
2. Measurement of T_{H} Activities. Spleen cell cultures derived from vaccinated animals are tested for recall to specific antigens by addition of either recombinant protein or peptide epitopes. Activation of T cells by such antigens, presented by accompanying splenic antigen presenting cells, APCs, is monitored by proliferation of these cultures or by cytokine production. The pattern of cytokine production also allows classification of TH response as type 1 or type 2. Because dominant T_{H}2 responses appear to correlate with the exclusion of cellular immunity in immunocompromised seropositive patients, it is possible to define the type of response engendered by a given PNV in patients, permitting manipulation of the resulting immune responses.
3. Delayed Type Hypersensitivity (DTH). DTH to viral antigen after i.d. injection is indicative of cellular, primarily MHC II-restricted, immunity. Because of the commercial availability of recombinant HIV proteins and synthetic peptides for known epitopes, DTH responses are easily determined in vaccinated vertebrates using these reagents, thus providing an additional in vivo correlate for inducing cellular immunity.

### Protection

Based upon the above immunologic studies, it is predictable that our vaccines are effective in vertebrates againsts challenge by virulent HIV. These studies are accomplished in an HIV_{IIIB}/chimpanzee challenge model after sufficient vaccination of these animals with a PNV construct, or a cocktail of PNV constructs comprised of gp160_{IIIB}, *gag*IIIB, nef_{IIIB} and *REV*_{IIIB}. The IIIB strain is useful in this regard as the chimpanzee titer of lethal doses of this strain has been established. However, the same studies are envisioned using any strain of HIV and the epitopes specific to or heterologous to the given strain. A second vaccination/challenge model, in addition to chimpanzees, is the *scid*-hu PBL mouse. This model allows testing of the human lymphocyte immune system and our vaccine with subsequent HIV challenge in a mouse host. This system is advantageous as it is easily adapted to use with any HIV strain and it provides evidence of protection against multiple strains of primary field isolates of HIV. A third challenge model utilizes hybrid HIV/SIV viruses (SHIV), some of which have been shown to infect rhesus monkeys and lead to immunodeficiency disease resulting in death [see Li, J., et al., J. AIDS 5:639-646, 1992]. Vaccination of rhesus with our polynucleotide vaccine constructs is protective against subsequent challenge with lethal doses of SHIV.

### PNV Construct Summary

HIV and other genes are ligated into an expression vector which has been optimized for polynucleotide vaccinations. Essentially all extraneous DNA is removed, leaving the essential elements of transcriptional promoter, immunogenic epitopes, transcriptional terminator, bacterial origin of replication and antibiotic resistance gene.

Expression of HIV late genes such as *env* and *gag* is *REV*-dependent and requires that the *REV* response element (RRE) be present on the viral gene transcript. A secreted form of gp120 can be generated in the absence of *REV* by substitution of the gp120 leader peptide with a heterologous leader such as from tPA (tissue-type plasminogen activator), and preferably by a leader peptide such as is found in highly expressed mammalian proteins such as immunoglobulin leader peptides. We have inserted a tPA-gp120 chimeric gene into V1Jns which efficiently expresses secreted gp120 in transfected cells (RD, a human rhabdomyosarcoma line). Monocistronic gp160 does not produce any protein upon transfection without the addition of a *REV* expression vector.

### Representative Construct Components Include (but are not restricted to):

1. tPA-gp120_{MN};
3. gp160_{IIIB};
10. *gag*IIIB: for anti-*gag* CTL;
11. tPA-gp 120_{IIIB};
12. gp160 with structural mutations: V1, V2, and/or V3 loop deletions or substitutions
20. Genes encoding antigens expressed by pathogens other than HIV, such as, but not limited to, influenza virus nucleoprotein, hemagglutinin, matrix, neuraminidase, and other antigenic proteins; herpes simplex virus genes; human papillomavirus genes; tuberculosis antigens; hepatitis A, B, or C virus antigens.

The protective efficacy of polynucleotide HIV immunogens against subsequent viral challenge is demonstrated by immunization with the non-replicating plasmid DNA of this invention. This is advantageous since no infectious agent is involved, assembly of virus particles is not required, and determinant selection is permitted. Furthermore, because the sequence of *gag* and protease and several of the other viral gene products is conserved among various strains of HIV, protection against subsequent challenge by a virulent strain of HIV that is homologous to, as well as strains heterologous to the strain from which the cloned gene is obtained, is enabled.

The i.m. injection of a DNA expression vector encoding gp160 results in the generation of significant protective immunity against subsequent viral challenge. In particular, gp160-specific antibodies and primary CTLs are produced. Immune responses directed against conserved proteins can be effective despite the antigenic shift and drift of the variable envelope proteins. Because each of the HIV gene products exhibit some degree of conservation, and because CTL are generated in response to intracellular expression and MHC processing, it is predictable that many virus genes give rise to responses analogous to that achieved for gp160. Thus, many of these genes have been cloned, as shown by the cloned and sequenced junctions in the expression vector (see below) such that these constructs are immunogenic agents in available form.

The invention offers a means to induce cross-strain protective immunity without the need for self-replicating agents or adjuvants. In addition, immunization with the instant polynucleotides offers a number of other advantages. This approach to vaccination should be applicable to tumors as well as infectious agents, since the CD8⁺ CTL response is important for both pathophysiological processes [K. Tanaka *et al*., Annu. Rev. Immunol. **6**, 359 (1988)]. Therefore, eliciting an immune response against a protein crucial to the transformation process may be an effective means of cancer protection or immunotherapy. The generation of high titer antibodies against expressed proteins after injection of viral protein and human growth hormone DNA suggests that this is a facile and highly effective means of making antibody-based vaccines, either separately or in combination with cytotoxic T-lymphocyte vaccines targeted towards conserved antigens.

The ease of producing and purifying DNA constructs compares favorably with traditional methods of protein purification, thus facilitating the generation of combination vaccines. Accordingly, multiple constructs, for example encoding gp 160, gp120, gp41, or any other HIV gene may be prepared, mixed and co-administered. Because protein expression is maintained following DNA injection, the persistence of B- and T-cell memory may be enhanced, thereby engendering long-lived humoral and cell-mediated immunity.

Standard techniques of molecular biology for preparing and purifying DNA constructs enable the preparation of the DNA immunogens of this invention. While standard techniques of molecular biology are therefore sufficient for the production of the products of this invention, the specific constructs disclosed herein provide novel polynucleotide immunogens which surprisingly produce cross-strain and primary HIV isolate neutralization, a result heretofore unattainable with standard inactivated whole virus or subunit protein vaccines.

The amount of expressible DNA or transcribed RNA to be introduced into a vaccine recipient will depend on the strength of the transcriptional and translational promoters used and on the immunogenicity of the expressed gene product. In general, an immunologically or prophylactically effective dose of about 1 ng to 100 mg, and preferably about 10 µg to 300 µg is administered directly into muscle tissue. Subcutaneous injection, intradermal introduction, impression through the skin, and other modes of administration such as intraperitoneal, intravenous, or inhalation delivery are also contemplated. It is also contemplated that booster vaccinations are to be provided. Following vaccination with HIV polynucleotide immunogen, boosting with HIV protein immunogens such as gp160, gp120, and *gag* gene products is also contemplated. Parenteral administration, such as intravenous, intramuscular, subcutaneous or other means of administration of interleukin-12 protein, concurrently with or subsequent to parenteral introduction of the PNV of this invention is also advantageous.

The polynucleotide may be naked, that is, unassociated with any proteins, adjuvants or other agents which impact on the recipients' immune sytem. In this case, it is desirable for the polycucleotide to be in a physiologically acceptable solution, such as, but not limited to, sterile saline or sterile buffered saline. Alternatively, the DNA may be associated with liposomes, such as lecithin liposomes or other liposomes known in the art, as a DNA-liposome mixture, or the DNA may be associated with an adjuvant known in the art to boost immune responses, such as a protein or other carrier. Agents which assist in the cellular uptake of DNA, such as, but not limited to, calcium ions, may also be used to advantage. These agents are generally referred to herein as transfection facilitating reagents and pharmaceutically acceptable carriers. Techniques for coating microprojectiles coated with polynucleotide are known in the art and are also useful in connection with this invention.

The following examples are offered by way of illustration and are not intended to limit the invention in any manner.

### EXAMPLE 1

### Materials descriptions

Vectors pF411 and pF412: These vectors were subcloned from vector pSP62 which was constructed in R. Gallo's lab. pSP62 is an available reagent from Biotech Research Laboratories, Inc. pSP62 has a 12.5 kb XbaI fragment of the HXB2 genome subcloned from lambda HXB2. SalI and Xba I digestion of pSP62 yields to HXB2 fragments: 5'-XbaI/SalI, 6.5 kb and 3'- SalI/XbaI, 6 kb. These inserts were subcloned into pUC 18 at SmaI and SalI sites yielding pF411 (5'-XbaI/SalI) and pF412 (3'-XbaI/SalI). pF411 contains *gag*/pol and pF412 contains tat/rev/*env*/nef.

### Repligen reagents:

recombinant *rev* (IIIB), #RP1024-10
rec. gp120 (IIIB), #RP1001-10
anti-*rev* monoclonal antibody, #RP1029-10
anti-gp120 mAB, #1C1, #RP1010-10

### AIDS Research and Reference Reagent Program: anti-gp41 mAB hybridoma, Chessie 8, #526

The strategies are designed to induce both cytotoxic T lymphocyte (CTL) and neutralizing antibody responses to HIV, principally directed at the HIV *gag* (~95% conserved) and *env* (gp160 or gp120; 70-80% conserved) gene products. gp160 contains the only known neutralizing antibody epitopes on the HIV particle while the importance of anti-*env* and anti-*gag* CTL responses are highlighted by the known association of the onset of these cellular immunities with clearance of primary viremia following infection, which occurs prior to the appearance of neutralizing antibodies, as well as a role for CTL in maintaining disease-free status. Because HIV is notorious for its genetic diversity, we hope to obtain greater breadth of neutralizing antibodies by including several representative *env* genes derived from clinical isolates and gp41 (~90% conserved), while the highly conserved *gag* gene should generate broad cross-strain CTL responses.

### EXAMPLE 2

### Heterologous Expression of HIV Late Gene Products

HIV structural genes such as *env* and *gag* require expression of the HIV regulatory gene, *rev,* in order to efficiently produce full-length proteins. We have found that *rev*-dependent expression of *gag* yielded low levels of protein and that *rev* itself may be toxic to cells. Although we achieved relatively high levels of *rev*-dependent expression of gp160 in vitro this vaccine elicited low levels of antibodies to gp160 following in vivo immunization with *rev*/gp160 DNA. This may result from known cytotoxic effects of *rev* as well as increased difficulty in obtaining *rev* function in myotubules containing hundreds of nuclei *(rev* protein needs to be in the same nucleus as a *rev*-dependent transcript for *gag* or *env* protein expression to occur). However, it has been possible to obtain *rev*-independent expression using selected modifications of the *env* gene.*gag*. Evaluation of these plasmids for vaccine purposes is underway.

In general, our vaccines have utilized primarily HIV (IIIB) *env* and *gag* genes for optimization of expression within our generalized vaccination vector, V1Jns, which is comprised of a CMV immediate-early (IE) promoter, BGH polyadenylation site, and a pUC backbone. Varying efficiencies, depending upon how large a gene segment is used (e.g., gp120 vs. gp160), of *rev*-independent expression may be achieved for *env* by replacing its native secretory leader peptide with that from the tissue-specific plasminogen activator (tPA) gene and expressing the resulting chimeric gene behind the CMVIE promoter with the CMV intron A. tPA-gp120 is an example of a secreted gp120 vector constructed in this fashion which functions well enough to elicit anti-gp120 immune responses in vaccinated mice and monkeys.

Because of reports that membrane-anchored proteins may induce much more substantial (and perhaps more specific for HIV neutralization) antibody responses compared to secreted proteins as well as to gain additional immune epitopes, we prepared V1Jns-tPA-gp160 and V lJns-rev/gp 160. The tPA-gp 160 vector produced detectable quantities of gp160 and gp120, without the addition of *rev,* as shown by immunoblot analysis of transfected cells, although levels of expression were much lower than that obtained for rev/gp 160, a *rev*-dependent gp160-expressing plasmid. This is probably because inhibitory regions (designated INS), which confer *rev* dependence upon the gp160 transcript, occur at multiple sites within gp160 including at the COOH-terminus of gp41 (see schematic below). A vector was prepared for a COOH-terminally truncated form of tPA-gp160, tPA-gp143, which was designed to increase the overall expression levels of *env* by elimination of these inhibitory sequences. The gp143 vector also eliminates intracellular gp41 regions containing peptide motifs (such as leu-leu) known to cause diversion of membrane proteins to the lysosomes rather than the cell surface. Thus, gp143 may be expected to increase both expression of the *env* protein (by decreasing *rev*-dependence) and the efficiency of transport of protein to the cell surface compared to full-length gp160 where these proteins may be better able to elicit anti-gp160 antibodies following DNA vaccination. tPA-gp143 was further modified by extensive silent mutagenesis of the *rev* response element (RRE) sequence (350 bp) to eliminate additional inhibitory sequences for expression. This construct, gp143/mutRRE, was prepared in two forms: either eliminating (form A) or retaining (form B) proteolytic cleavage sites for gp120/41. Both forms were prepared because of literature reports that vaccination of mice using uncleavable gp160 expressed in vaccinia elicited much higher levels of antibodies to gp160 than did cleavable forms.

A quantitative ELISA for gp160/gp120 expression in cell transfectants was developed to determine the relative expression capabilities for these vectors. In vitro transfection of 293 cells followed by quantification of cell-associated vs. secreted/released gp120 yielded the following results: (1) tPA-gp160 expressed 5-10X less gp 120 than *rev*/gp160 with similar proportions retained intracellularly vs. trafficked to the cell surface; (2) tPA-gp143 gave 3-6X greater secretion of gp120 than *rev*/gp160 with only low levels of cell-associated gp143, confirming that the cytoplasmic tail of gp160 causes intracellular retention of gp160 which can be overcome by partial deletion of this sequence; and, (3) tPA-gp143/mutRRE A and B gave ~10X greater expression levels of protein than did parental tPA-gp143 while elimination of proteolytic processing was confirmed for form A.

Thus, our strategy to increase *rev*-independent expression has yielded stepwise increases in overall expression as well as redirecting membrane-anchored gp143 to the cell surface away from lysosomes. It is important to note that it should be possible to insert gp120 sequences derived from various viral isolates within a vector cassette containing these modifications which reside either at the NH₂-terminus (tPA leader) or COOH-terminus (gp41), where few antigenic differences exist between different viral strains. In other words, this is a generic construct which can easily be modified by inserting gp120 derived from various primary viral isolates to obtain clinically relevant vaccines.

To apply these expression strategies to viruses that are relevant for vaccine purposes and confirm the generality of our approaches, we also prepared a tPA-gp120 vector derived from a primary HIV isolate (containing the North American concensus V3 peptide loop; macrophage-tropic and nonsyncytia-inducing phenotypes). This vector gave high expression/secretion of gp120 with transfected 293 cells and elicited anti-gp120 antibodies in mice demonstrating that it was cloned in a functional form. Primary isolate gp160 genes will also be used for expression in the same way as for gp160 derived from laboratory strains.

### EXAMPLE 3

### Immune Responses to HIV-1 env Polynucleotide Vaccines:

African green (AGM) and Rhesus (RHM) monkeys which received gp120 DNA vaccines showed low levels of neutralizing antibodies following 2-3 vaccinations, which could not be increased by additional vaccination. These results, as well as increasing awareness within the HIV vaccine field that oligomeric gp160 is probably a more relevant target antigen for eliciting neutralizing antibodies than gp120 monomers (Moore and Ho, J. Virol. *67*: 863 (1993)), have led us to focus upon obtaining effective expression of gp160-based vectors (see above). Mice and AGM were also vaccinated with the primary isolate derived tPA-gp120 vaccine. These animals exhibited anti-V3 peptide (using homologous sequence) reciprocal endpoint antibody titers ranging from 500-5000, demonstrating that this vaccine design is functional for clinically relevant viral isolates.

The gp160-based vaccines, *rev*-gp160 and tPA-gp160, failed to consistently elicit antibody responses in mice and nonhuman primates or yielded low antibody titers. Our initial results with the tPA-gp143 plasmid yielded geometric mean titers > 10³ in mice and AGM following two vaccinations. These data indicate that we have signficantly improved the immunogenicity of gp160-like vaccines by increasing expression levels. This construct, as well as the tPA-gp143/mutRRE A and B vectors, will continue to be characterized for antibody responses, especially for virus neutralization.

Significantly, gp 120 DNA vaccination produced potent helper T cell responses in all lymphatic compartments tested (spleen, blood, inguinal, mesenteric, and iliac nodes) with T_{H}1-like cytokine secretion profiles (i.e., g-interferon and IL-2 production with little or no IL-4). These cytokines generally promote strong cellular immunity and have been associated with maintenance of a disease-free state for HIV-seropositive patients. Lymph nodes have been shown to be primary sites for HIV replication, harboring large reservoirs of virus even when virus cannot be readily detected in the blood. A vaccine which can elicit anti-HIV immune responses at a variety of lymph sites, such as we have shown with our DNA vaccine, may help prevent successful colonization of the lymphatics following initial infection.

As stated previously, we consider realization of the following objectives to be essential to maximize our chances for success with this program: (1) *env*-based vectors capable of generating stronger neutralizing antibody responses in primates; (2) *gag* and *env* vectors which elicit strong T-lymphocyte responses as characterized by CTL and helper effector functions in primates; (3) use of *env* and *gag* genes from clinically relevant HIV-1 strains in our vaccines and characterization of the immunologic responses, especially neutralization of primary isolates, they elicit; (4) demonstration of protection in an animal challenge model such as chimpanzee/HIV (IIIB) or rhesus/SHIV using appropriate optimized vaccines; and, (5) determination of the duration of immune responses appropriate to clinical use. Significant progress has been made on the first three of these objectives and experiments are in progress to determine whether our recent vaccination constructs for gp160 and *gag* will improve upon these initial results.

### EXAMPLE 4

### Vectors For Vaccine Production

### A. V1Jneo EXPRESSION VECTOR:

It was necessary to remove the amp^{r} gene used for antibiotic selection of bacteria harboring V1J because ampicillin may not be used in large-scale fermenters. The amp^{r} gene from the pUC backbone of V1J was removed by digestion with SspI and Earn 1105I restriction enzymes. The remaining plasmid was purified by agarose gel electrophoresis, blunt-ended with T4 DNA polymerase, and then treated with calf intestinal alkaline phosphatase. The commercially available kan^{r} gene, derived from transposon 903 and contained within the pUC4K plasmid, was excised using the PstI restriction enzyme, purified by agarose gel electrophoresis, and blunt-ended with T4 DNA polymerase. This fragment was ligated with the V1J backbone and plasmids with the kan^{r} gene in either orientation were derived which were designated as V1Jneo #'s 1 and 3. Each of these plasmids was confirmed by restriction enzyme digestion analysis, DNA sequencing of the junction regions, and was shown to produce similar quantities of plasmid as V1J. Expression of heterologous gene products was also comparable to V1J for these V1Jneo vectors. We arbitrarily selected V1Jneo#3, referred to as V1Jneo hereafter, which contains the kan^{r} gene in the same orientation as the amp^{r} gene in V1J as the expression construct.

### B. VIJns Expression Vector:

An Sfi I site was added to V1Jneo to facilitate integration studies. A commercially available 13 base pair Sfi I linker (New England BioLabs) was added at the Kpn I site within the BGH sequence of the vector. V1Jneo was linearized with Kpn I, gel purified, blunted by T4 DNA polymerase, and ligated to the blunt Sfi I linker. Clonal isolates were chosen by restriction mapping and verified by sequencing through the linker. The new vector was designated V1Jns. Expression of heterologous genes in V1Jns (with Sfi I) was comparable to expression of the same genes in V1Jneo (with Kpn I).

### C. V1Jns-tPA:

In order to provide an heterologous leader peptide sequence to secreted and/or membrane proteins, V1Jn was modified to include the human tissue-specific plasminogen activator (tPA) leader. Two synthetic complementary oligomers were annealed and then ligated into V1Jn which had been BgIII digested. The sense and antisense oligomers were 5'-GATC ACC ATG GAT GCA ATG AAG AGA GGG CTC TGC TGT GTG CTG CTG CTG TGT GGA GCA GTC TTC GTT TCG CCC AGC GA-3', SEQ.ID:18:, and 5'-GAT CTC GCT GGG CGA AAC GAA GAC TGC TCC ACA CAG CAG CAG CAC ACA GCA GAG CCC TCT CTT CAT TGC ATC CAT GGT-3'. The Kozak sequence is underlined in the sense oligomer. These oligomers have overhanging bases compatible for ligation to BglII-cleaved sequences. After ligation the upstream BglII site is destroyed while the downstream BglII is retained for subsequent ligations. Both the junction sites as well as the entire tPA leader sequence were verified by DNA sequencing. Additionally, in order to conform with our consensus optimized vector V1Jns (=V1Jneo with an SfiI site), an SfiI restriction site was placed at the KpnI site within the BGH terminator region of V1Jn-tPA by blunting the KpnI site with T4 DNA polymerase followed by ligation with an SfiI linker (catalogue #1138, New England Biolabs). This modification was verified by restriction digestion and agarose gel electrophoresis.

### EXAMPLE 5

### I. HIV env Vaccine Constructs:

### Vaccines Producing Secreted env-derived Antigen (gp120 and gp140):

Expression of the *REV* -dependent *env* gene as gp 120 was conducted as follows: gp 120 was PCR-cloned from the MN strain of HIV with either the native leader peptide sequence (V1Jns-gp120), or as a fusion with the tissue-plasminogen activator (tPA) leader peptide replacing the native leader peptide (V1Jns-tPA-gp120). tPA-gp120 expression has been shown to be *REV*-independent [B.S. Chapman *et al*., Nuc. Acids Res. **19,** 3979 (1991); it should be noted that other leader sequences would provide a similar function in rendering the gp120 gene *REV* independent]. This was accomplished by preparing the following gp120 constructs utilizing the above described vectors:

### EXAMPLE 6

### gp120 Vaccine Constructs:

### A. V1Jns-tPA-HIV_{MN} gp120:

HIV_{MN} gp120 gene (Medimmune) was PCR-amplified using oligomers designed to remove the first 30 amino acids of the peptide leader sequence and to facilitate cloning into V1Jns-tPA creating a chimeric protein consisting of the tPA leader peptide followed by the remaining gp120 sequence following amino acid residue 30. This design allows for *REV* -independent gp120 expression and secretion of soluble gp120 from cells harboring this plasmid. The sense and antisense PCR oligomers used were 5'-CCC CGG ATC CTG ATC ACA GAA AAA TTG TGGGTC ACA GTC-3', and 5'-C CCC AGG AATC CAC CTG TTA GCG CTT TTC TCT CTG CAC CAC TCT TCT C-3'. The translation stop codon is underlined. These oligomers contain BamHI restriction enzyme sites at either end of the translation open reading frame with a BclI site located 3' to the BamHI of the sense oligomer. The PCR product was sequentially digested with BclI followed by BamHI and ligated into V1Jns-tPA which had been BglII digested followed by calf intestinal alkaline phosphatase treatment. The resulting vector was sequenced to confirm inframe fusion between the tPA leader and gp120 coding sequence, and gp120 expression and secretion was verified by immunoblot analysis of transfected RB cells.

### B. V1Jns-tPA-HIV_{IIIB} gp120:

This vector is analogous to I.A. except that the HIV IIIB strain was used for gp120 sequence. The sense and antisense PCR oligomers used were: 5'-GGT ACA TGA TCA CA GAA AAA TTG TGG GTC ACA GTC-3', and 5'-CCA CAT TGA TCA GAT ATC TTA TCT TTT TTC TCT CTG CAC CAC TCT TC-3', respectively. These oligomers provide BclI sites at either end of the insert as well as an EcoRV just upstream of the BclI site at the 3'-end. The 5'-terminal BclI site allows ligation into the BglII site of V1Jns-tPA to create a chimeric tPA-gp120 gene encoding the tPA leader sequence and gp 120 without its native leader sequence. Ligation products were verified by restriction digestion and DNA sequencing.

### EXAMPLE 7

### gp140 Vaccine Constructs:

These constructs was prepared by PCR similarly as tPA-gp120 with the tPA leader in place of the native leader, but designed to produce secreted antigen by terminating the gene immediately NH₂-terminal of the transmembrane peptide (projected carboxyterminal amino acid sequence = NH₂-.....TNWLWYIK-COOH). Unlike the gp120-producing constructs, gp140 constructs should produce oligomeric antigen and retain known gp41-contained antibody neutralization epitopes such as ELDKWA defined by the 2F5 monoclonal antibody.

Constructs were prepared in two forms (A or B) depending upon whether the gp160 proteolytic cleavage sites at the junction of gp120 and gp41 were retained (B) or eliminated (A) by appropriate amino acid substitutions as described by Kieny et al., (Prot. Eng. 2: 219-255 (1988)) (wild type sequence = NH₂-...KAKRRVVQREKR...COOH and the mutated sequence = NH₂-...KAQNHVVQNEHQ...COOH with mutated amino acids underlined).

### A. V1Jns-tPA-gp140/mutRRE-A/SRV-1 3'-UTR (based on HIV-1_{IIIB}):

This construct was obtained by PCR using the following sense and antisense PCR oligomers: 5'-CT GAA AGA CCA GCA ACT CCT AGG GAAT TTG GGG TTG CTC TGG-3', SEQ.ID: :, and 5'-CGC AGG GGA GGT GGT CTA GAT ATC TTA TTA TTT TAT ATA CCA CAG CCA ATT TGT TAT G-3' to obtain an AvrII/EcoRV segment from vector IVB (containing the optimized RRE-A segment). The 3'-UTR, prepared as a synthetic gene segment, that is derived from the Simian Retrovirus-1 (SRV-1, see below) was inserted into an SrfI restriction enzyme site introduced immediately 3'- of the gp140 open reading frame. This UTR sequence has been described previously as facilitating *rev*-independent expression of HIV *env* and *gag*.

### B. V1Jns-tPA-gp140/mutRRE-B/SRV-1 3'-UTR (based on HIV-1_{IIIB}):

This construct is similar to IIA except that the *env* proteolytic cleavage sites have been retained by using construct IVC as starting material.

### C. V1Jns-tPA-gp140/opt30-A (based on HIV-1_{IIIB}):

This construct was derived from IVB by AvrII and Srfl restriction enzyme digestion followed by ligation of a synthetic DNA segment corresponding to gp30 but comprised of optimal codons for translation (see gp32-opt below). The gp30-opt DNA was obtained from gp32-opt by PCR amplification using the following sense and antisense oligomers: 5'-GGT ACA CCT AGG CAT CTG GGG CTG CTC TGG-3'; and, 5'-CCA CAT GAT ATC G CCC GGG C TTA TTA TTT GAT GTA CCA CAG CCA GTT GGT GAT G-3', respectively. This DNA segment was digested with AvrII and EcoRV restriction enzymes and ligated into V 1Jns-tPA-gp143/opt32-A (IVD) that had been digested with AvrII and SrfI to remove the corresponding DNA segment. The resulting products were verified by DNA sequencing of ligation junctions and immunoblot analysis.

### D. V1Jns-tPA-gp140/opt30-B (based on HIV-1_{IIIB}):

This construct is similar to IIC except that the *env* proteolytic cleavage sites have been retained.

### E. V1Jns-tPA-gp140/opt all-A:

The *env* gene of this construct is comprised completely of optimal codons. The constant regions (C1, C5, gp32) are those described in IVB,D,H with an additional synthetic DNA segment corresponding to variable regions 1-5 is inserted using a synthetic DNA segment comprised of optimal codons for translation (see example below based on HIV-1 MN V1-V5).

### F. V1Jns-tPA-gp140/opt all-B:

This construct is similar to IIE except that the *env* proteolytic cleavage sites have been retained.

### G. V1Jns-tPA-gp140/opt all-A (non-IIIB strains):

This construct is similar to IIE above except that *env* amino acid sequences from strains other than IIIB are used to determine optimum codon useage throughout the variable (V1-V5) regions.

### H. V1Jns-tPA-gp140/opt all-B (non-IIIB strains):

This construct is similar to IIG except that the *env* proteolytic cleavage sites have been retained.

### EXAMPLE 8

### gp160 Vaccine Constructs:

Constructs were prepared in two forms (A or B) depending upon whether the gp160 proteolytic cleavage sites as described above.

### A. V1Jns-rev/env:

This vector is a variation of the one described in section D above except that the entire *tat* coding region in exon 1 is deleted up to the beginning of the *REV* open reading frame. V1Jns-gp160_{IIIB} (see section A. above) was digested with PstI and KpnI restriction enzymes to remove the 5'-region of the gp160 gene. PCR amplification was used to obtain a DNA segment encoding the first*REV* exon up to the KpnI site in gp160 from the HXB2 genomic clone. The sense and antisense PCR oligomers were 5'-GGT ACA CTG CAG TCA CCG TCC T ATG GCA GGA AGA AGC GGA GAC-3', and 5'-CCA CAT CA GGT ACC CCA TAA TAG ACT GTG ACC-3', respectively. These oligomers provide PstI and KpnI restriction enzyme sites at the 5'- and 3'- termini of the DNA fragment, respectively. The resulting DNA was digested with PstI and KpnI, purified from an agarose electrophoretic gel, and ligated with V1Jns-gp160(PstI/KpnI). The resulting plasmid was verified by restriction enzyme digestion.

### B. V1Jns-gp160:

HIV_{IIIb} gp160 was cloned by PCR amplification from plasmid pF412 which contains the 3'-terminal half of the HIV_{IIIb} genome derived from HIV_{IIIb} clone HXB2. The PCR sense and antisense oligomers were 5'-GGT ACA TGA TCA ACC ATG AGA GTG AAG GAG AAA TAT CAG C-3',, and 5'-CCA CAT TGA TCA GAT ATC CCC ATC TTA TAG CAA AAT CCT TTC C-3',, respectively. The Kozak sequence and translation stop codon are underlined. These oligomers provide BclI restriction enzyme sites outside of the translation open reading frame at both ends of the *env* gene. (BclI-digested sites are compatible for ligation with BglII-digested sites with subsequent loss of sensitivity to both restriction enzymes. BclI was chosen for PCR-cloning gp160 because this gene contains internal BglII and as well as BamHI sites). The antisense oligomer also inserts an EcoRV site just prior to the BclI site as described above for other PCR-derived genes. The amplified gp160 gene was agarose gel-purified, digested with BclI, and ligated to V1Jns which had been digested with BglII and treated with calf intestinal alkaline phosphatase. The cloned gene was about 2.6 kb in size and each junction of gp160 with V1Jns was confirmed by DNA sequencing.

### C. V1Jns-tPA-gp160 (based on HIV-1_{IIIB}):

This vector is similar to Example 1 (C) above, except that the full-length gp160, without the native leader sequence, was obtained by PCR. The sense oligomer was the same as used in I.C. and the antisense oligomer was 5'-CCA CAT TGA TCA GAT ATC CCC ATC TTA TAG CAA AAT CCT TTC C-3'. These oligomers provide BclI sites at either end of the insert as well as an EcoRV just upstream of the BclI site at the 3'-end. The 5'-terminal BclI site allows ligation into the BglII site of V1Jns-tPA to create a chimeric tPA-gp160 gene encoding the tPA leader sequence and gp160 without its native leader sequence. Ligation products were verified by restriction digestion and DNA sequencing.

### D. V1Jns-tPA-gp160/opt C1/opt41-A (based on HIV-1_{IIIB}):

This construct was based on IVH, having a complete optimized codon segment for C5 and gp41, rather than gp32, with an additional optimized codon segment (see below) replacing C1 at the amino terminus of gp120 following the tPA leader. The new C 1 segment was joined to the remaining gp143 segment via SOE PCR using the following oligomers for PCR to synthesize the joined C1/143 segment: 5'-CCT GTG TGT GAG TTT AAA C TGC ACT GAT TTG AAG AAT GAT ACT AAT AC-3'. The resulting gp143 gene contains optimal codon useage except for V1-V5 regions and has a unique PmeI restriction enzyme site placed at the junction of C1 and V 1 for insertion of variable regions from other HIV genes.

### E. V1Jns-tPA-gp160/opt C1/opt41-B (based on HIV-1_{IIIB}):

This construct is similar to IIID except that the *env* proteolytic cleavage sites have been retained.

### F V1Jns-tPA-gp160/opt all-A (based on HIV-1_{IIIB}):

The *env* gene of this construct is comprised completetly of optimal codons as described above. The constant regions (C1, C5, gp32) are those described in IIID,E which is used as a cassette (employed for all completely optimized gp160s) while the variable regions, V1-V5, are derived from a synthetic DNA segment comprised of optimal codons.

### G. V1Jns-tPA-gp160/opt all-B:

This construct is similar to IIIF except that the *env* proteolytic cleavage sites have been retained.

### H. V1Jns-tPA-gp160/opt all-A (non-IIIB strains):

This construct is similar to IIIF above except that *env* amino acid sequences from strains other than IIIB were used to determine optimum codon useage throughout the variable (V1-V5) regions.

### I. V1Jns-tPA-gp160/opt all-B (non-IIIB strains):

This construct is similar to IIIH except that the *env* proteolytic cleavage sites have been retained.

### EXAMPLE 9

### gp143 Vaccine Constructs:

These constructs were prepared by PCR similarly as other tPA-containing constructs described above (tPA-gp120, tPA-gp140, and tPA-gp160), with the tPA leader in place of the native leader, but designed to produce COOH-terminated, membrane-bound *env* (projected intracellular amino acid sequence= NH₂-NRVRQGYSP-COOH). This construct was designed with the purpose of combining the increased expression of *env* accompanying tPA introduction and minimizing the possibility that a transcript or peptide region corresponding to the intracellular portion of *env* might negatively impact expression or protein stability/transport to the cell surface. Constructs were prepared in two forms (A or B) depending upon whether the gp160 proteolytic cleavage sites were removed or retained as described above. The residual gp41 fragment resulting from truncation to gp143 is referred to as gp32.

### A. V1Jns-tPA-gp143:

This construct was prepared by PCR using plasmid pF412 with the following sense and antisense PCR oligomers: 5'-GGT ACA TGA TCA CA GAA AAA TTG TGG GTC ACA GTC-3', SEQ.ID: :, and 5'- CCA CAT TGA TCA G CCC GGG C TTA GGG TGA ATA GCC CTG CCT CAC TCT GTT CAC-3'. The resulting DNA segment contains BclI restriction sites at either end for cloning into V1Jns-tPA/BglII-digested with an SrfI site located immediately 3'- to the *env* open reading frame. Constructs were verified by DNA sequencing of ligation junctions and immunoblot analysis of transfected cells.

### B. V1Jns-tPA-gp143/mutRRE-A:

This construct was based on IVA by excising the DNA segment using the unique MunI restriction enzyme site and the downstream SrfI site described above. This segment corresponds to a portion of the gp120 C5 domain and the entirety of gp32. A synthetic DNA segment corresponding to ~350 bp of the *rev* response element (RRE A) of gp160,comprised of optimal codons for translation, was joined to the remaining gp32 segment by splice overlap extension (SOE) PCR creating an AvrII restriction enzyme site at the junction of the two segments (but no changes in amino acid sequence). These PCR reactions were performed using the following sense and antisense PCR oligomers for generating the gp32-containing domain: 5'-CT GAA AGA CCA GCA ACT CCT AGG GAT TTG GGG TTG CTG TGG-3' and 5'-CCA CAT TGA TCA G CCC GGG C TTA GGG TGA ATA GCC CTG CCT CAC TCT GTT CAC-3' (which was used as the antisense oligomer for IVA), respectively. The mutated RRE (mutRRE-A) segment was joined to the wild type sequence of gp32 by SOE PCR using the following sense oligomer, 5'-GGT ACA CAA TTG GAG GAG CGA GTT ATA TAA ATA TAA G-3', and the antisense oligomer used to make the gp32 segment. The resulting joined DNA segment was digested with MunI and SrfI restriction enzymes and ligated into the parent gp143/MunI/SrfI digested plasmid. The resulting construct was verified by DNA sequencing of ligation and SOE PCR junctions and immunoblot analysis of transfected cells.

### C. V1Jns-tPA-gp143/mutRRE-B:

This construct is similar to IVB except that the *env* proteolytic cleavage sites have been retained by using the mutRRE-B synthetic gene segment in place of mutRRE-A.

### D. V1Jns-tPA-gp143/opt32-A:

This construct was derived from IVB by AvrIl and Srfl restriction enzyme digestion followed by ligation of a synthetic DNA segment corresponding to gp32 but comprised of optimal codons for translation (see gp32 opt below). The resulting products were verified by DNA sequencing of ligation junctions and immunoblot analysis.

### E. V1Jns-tPA-gp143/opt32-B:

This construct is similar to IVD except that the *env* proteolytic cleavage sites have been retained by using IVC as the initial plasmid.

### G. V1Jns-tPA-gp143/SRV-1 3'-UTR:

This construct is similar to IVA except that the 3'-UTR derived from the Simian Retrovirus-1 (SRV-1, see below) was inserted into the SrfI restriction enzyme site introduced immediately 3'- of the gp143 open reading frame. This UTR sequence has been described previously as facilitating *rev*-independent expression of HIV *env* and *gag*.

### H. V1Jns-tPA-gp143/opt C1/opt32A:

This construct was based on IVD, having a complete optimized codon segment for C5 and gp32 with an additional optimized codon segment (see below) replacing C 1 at the amino terminus of gp 120 following the tPA leader. The new C1 segment was joined to the remaining gp143 segment via SOE PCR using the following oligomers for PCR to synthesize the joined C1/143 segment: 5'-CCT GTG TGT GAG TTT AAA C TGC ACT GAT TTG AAG AAT GAT ACT AAT AC-3'. The resulting gp143 gene contains optimal codon useage except for V1-V5 regions and has a unique PmeI restriction enzyme site placed at the junction of C1 and V 1 for insertion of variable regions from other HIV genes.

### I. V1Jns-tPA-gp143/opt C1/opt32B:

This construct is similar to IVH except that the *env* proteolytic cleavage sites have been retained.

### J. V1Jns-tPA-gp143/opt all-A:

The *env* gene of this construct is comprised completely of optimal codons. The constant regions (C1, C5, gp32) are those described in 4B,D,H with an additional synthetic DNA segment corresponding to variable regions V1-V5 is inserted using a synthetic DNA segment comprised of optimal codons for translation.

### K. V1Jns-tPA-gp143/opt all-B:

This construct is similar to IVJ except that the *env* proteolytic cleavage sites have been retained.

### L. V1Jns-tPA-gp143/opt all-A (non-IIIB strains):

This construct is similar to IIIG above except that *env* amino acid sequences from strains other than IIIB were used to determine optimum codon useage throughout the variable (V1-V5) regions.

### M. V1Jns-tPA-gp143/opt all-B (non-IIIB strains):

This construct is similar to IIIG above except that *env* amino acid sequences from strains other than IIIB were used to determine optimum codon useage throughout the variable (V1-V5) regions.

### EXAMPLE 10

### gp143/glyB Vaccine Constructs:

These constructs were prepared by PCR similarly as other tPA-containing constructs described above (tPA-gp120, tPA-gp140, tPA-gp143 and tPA-gp160), with the tPA leader in place of the native leader, but designed to produce COOH-terminated, membrane-bound *env* as with gp143. However, gp143/glyB constructs differ from gp143 in that of the six amino acids projected to comprise the intracellular peptide domain, the last 4 are the same those at the carboxyl terminus of human glycophorin B (glyB) protein (projected intracellular amino acid sequence= NH₂-NRLIKA-COOH with the underlined residues corresponding to glyB and "R" common to both *env* and glyB). This construct was designed with the purpose gaining additional *env* expression and directed targeting to the cell surface by completely eliminating any transcript or peptide region corresponding to the intracellular portion of *env* that might negatively impact expression or protein stability/transport to the cell surface by replacing this region with a peptide sequence from an abundantly expressed protein (glyB) having a short cytoplasmic domain (intracellular amino acid sequence= NH₂-RRLIKA-COOH). Constructs were prepared in two forms (A or B) depending upon whether the gp160 proteolytic cleavage sites were removed or retained as described above.

### A. V1Jns-tPA-gp143/opt32-A/glyB:

This construct is the same as IVD except that the following antisense PCR oligomer was used to replace the intracellular peptide domain of gp143 with that of glycophorin B as described above: 5'-CCA CAT GAT ATC G CCC GGG C TTA TTA GGC CTT GAT CAG CCG GTT CAC AAT GGA CAG CAC AGC-3'.

### B. V1Jns-tPA-gp143/opt32-B/glyB:

This construct is similar to VA except that the *env* proteolytic cleavage sites have been retained.

### C. V1Jns-tPA-gp143/opt C1/opt32-A/glyB:

This construct is the same as VA except that the first constant region (C1) of gp120 is replaced by optimal codons for translation as with IVH.

### D. V1Jns-tPA-gp143/opt C 1/opt32-B/glyB:

This construct is similar to VC except that the *env* proteolytic cleavage sites have been retained.

### E. V1Jns-tPA-gp143/opt all-A/glyB:

The *env* gene of this construct is comprised completetly of optimal codons as described above.

### F. V1Jns-tPA-gp143/opt all-B/glyB:

This construct is similar to VE except that the *env* proteolytic cleavage sites have been retained.

### G. V1Jns-tPA-gp143/opt all-A/glyB (non-IIIB strains):

This construct is similar to IIIG above except that *env* amino acid sequences from strains other than IIIB were used to determine optimum codon useage throughout the variable (V1-V5) regions.

### H. V1Jns-tPA-gp143/opt all-B/glyB (non-IIIB strains):

This construct is similar to VG except that the *env* proteolytic cleavage sites have been retained.

### HIV env Vaccine Constructs with Variable Loop Deletions:

These constructs may include all *env* forms listed above (gp120, gp140, gp143, gp 160, gp143/glyB) but have had variable loops within the gp 120 region deleted during preparation (*e*.*g*., V1, V2, and/or V3). The purpose of these modifications is to eliminate peptide segments which may occlude exposure of conserved neutralization epitopes such as the CD4 binding site. For example, the following oligomer was used in a PCR reaction to create a V1/V2 deletion resulting in adjoining THE C1 and C2 segments: 5'-CTG ACC CCC CTG TGT GTG GGG GCT GGC AGT TGT AAC ACC TCA GTC ATT ACA CAG-3'.

### EXAMPLE 11

### Design of Synthetic Gene Segments for Increased env Gene Expression:

Gene segments were converted to sequences having identical translated sequences (except where noted) but with alternative codon usage as defined by R. Lathe in a research article from *J. Molec. Biol*. Vol. 183, pp. 1-12 (1985) entitled "Synthetic Oligonucleotide Probes Deduced from Amino Acid Sequence Data: Theoretical and Practical Considerations". The methodology described below to increase *rev*-independent expression of HIV *env* gene segments was based on our hypothesis that the known inability to express this gene efficiently in mammalian cells is a consequence of the overall transcript composition. Thus, using alternative codons encoding the same protein sequence may remove the constraints on *env* expression in the absence of *rev*. Inspection of the codon usage within *env* revealed that a high percentage of codons were among those infrequently used by highly expressed human genes. The specific codon replacement method employed may be described as follows employing data from Lathe et al.:
1. Identify placement of codons for proper open reading frame.
2. Compare wild type codon for observed frequency of use by human genes (refer to Table 3 in Lathe et al.).
3. If codon is not the most commonly employed, replace it with an optimal codon for high expression based on data in Table 5.
4. Inspect the third nucleotide of the new codon and the first nucleotide of the adjacent codon immediately 3'- of the first. If a 5'-CG-3' pairing has been created by the new codon selection, replace it with the choice indicated in Table 5.
5. Repeat this procedure until the entire gene segment has been replaced.
6. Inspect new gene sequence for undesired sequences generated by these codon replacements (e.g., "ATTTA" sequences, inadvertent creation of intron splice recognition sites, unwanted restriction enzyme sites, etc.) and substitute codons that eliminate these sequences.
7. Assemble synthetic gene segments and test for improved expression.
These methods were used to create the following synthetic gene segments for HIV *env* creating a gene comprised entirely of optimal codon usage for expression: (i) gp120-C1 (opt); (ii) V1-V5 (opt); (iii) RRE-A/B (mut or opt); and (iv) gp30 (opt) with percentages of codon replacements/nucleotide substitutions of 56/19, 73/26, 78/28, and 61/25 obtained for each segment, respectively. Each of these segments has been described in detail above with actual sequences listed below.

### gp120-C1 (opt)

This is a gp120 constant region 1 (C1) gene segment from the mature N-terminus to the beginning of V 1 designed to have optimal codon usage for expression.

  1  TGATCACAGA GAAGCTGTGG GTGACAGTGT ATTATGGCGT GCCAGTCTGG

 51  AAGGAGGCCA CCACCACCCT GTTCTGTGCC TCTGATGCCA AGGCCTATGA

101  CACAGAGGTG CACAATGTGT GGGCCACCCA TGCCTGTGTG CCCACAGACC

151  CCAACCCCCA GGAGGTGGTG CTGGTGAATG TGACTGAGAA CTTCAACATG

201  TGGAAGAACA ACATGGTGGA GCAGATGCAT GAGGACATCA TCAGCCTGTG

251  GGACCAGAGC CTGAAGCCCT GTGTGAAGCT GACCCCCCTG TGTGTGAGTT

301  TAAAC

### MN V1-V5 (opt)

This is a gene segment corresponding to the derived protein sequence for HIV MN V1-V5 (1066BP) having optimal codon usage for expression.

1 AGTTTAAACT GCACAGACCT GAGGAACACC ACCAACACCA ACAACTCCAC

51 AGCCAACAAC AACTCCAACT CCGAGGGCAC CATCAAGGGG GGGGAGATGA

101 AGAACTGCTC CTTCAACATC ACCACCTCCA TCAGGGACAA GATGCAGAAG

151 GAGTATGCCC TGCTGTACAA GCTGGACATT GTGTCCATTG ACAATGACTC

201 CACCTCCTAC AGGCTGATCT CCTGCAACAC CTCTGTCATC ACCCAGGCCT

251 GCCCCAAAAT CTCCTTTGAG CCCATCCCCA TCCACTACTG TGCCCCTGCT

301 GGCTTTGCCA TCCTGAAGTG CAATGACAAG AAGTTCTCTG GCAAGGGCTC

351 CTGCAAGAAT GTGTCCACAG TGCAGTGCAC ACATGGCATC AGGCCTGTGG

401 TGTCCACCCA GCTGCTGCTG AATGGCTCCC TGGCTGAGGA GGAGGTGGTC

451 ATCAGGTCTG AGAACTTCAC AGACAATGCC AAGACCATCA TCGTGCACCT

501 GAATGAGTCT GTGCAGATCA ACTGCACCAG GCCCAACTAC AACAAGAGGA

551 AGAGGATCCA CATTGGCCCT GGCAGGGCCT TCTACACCAC CAAGAACATC

601 ATTGGCACCA TCAGGCAGGC CCACTGCAAC ATCTCCAGGG CCAAGTGGAA

651 TGACACCCTG AGGCAGATTG TGTCCAAGCT GAAGGAGCAG TTCAAGAACA

701 AGACCATTGT GTTCAACCAG TCCTCTGGGG GGGACCCTGA GATTGTGATG

751 CACTCCTTCA ACTGTGGGGG GGAGTTCTTC TACTGCAACA CCTCCCCCCT

801 GTTCAACTCC ACCTGGAATG GCAACAACAC CTGGAACAAC ACCACAGGCT

851 CCAACAACAA CATCACCCTC CAGTGCAAGA TCAAGCAGAT CATCAACATG

901 TGGCAGGAGG TGGGCAAGGC CATGTATGCC CCCCCCATTG AGGGCCAGAT

951 CAGGTGCTCC TCCAACATCA CAGGCCTGCT GCTGACCAGG GATGGGGGGA

1001 AGGACACAGA CACCAACGAC ACCGAAATCT TCAGGCCTGG GGGGGGGGAC

1051 ATGAGGGACA ATTGG

### RRE.Mut (A)

This is a DNA segment corresponding to the *rev* response element (RRE) of HIV-1 comprised of optimal codon usage for expression. The "A" form also has removed the known proteolytic cleavage sites at the gp120/gp41 junction by using the nucleotides indicated in boldface.

1 GACAATTGGA GGAGCGAGTT ATATAAATAT AAGGTGGTGA AGATTGAGCC

51 CCTGGGGGTG GCCCCAACAA AAGCTCAGAACCACGTGGTG CAGAACGAGC

101 ACCAGGCCGT GGGCATTGGG GCCCTGTTTC TGGGCTTTCT GGGGGCTGCT

151 GGCTCCACAA TGGGCGCCGC TAGCATGACC CTCACCGTGC AAGCTCGCCA

201 GCTGCTGAGT GGCATCGTCC AGCAGCAGAA CAACCTGCTC CGCGCCATCG

251 AAGCCCAGCA GCACCTCCTC CAGCTGACTG TGTGGGGGAT CAAACAGCTT

301 CAGGCCCGGG TGCTGGCCGT CGAGCGCTAT CTGAAAGACC AGCAACTCCT

351 AGGC

### RRE.Mut (B)

This is a DNA segment corresponding to the *rev* response element (RRE) of HIV-1 comprised of optimal codon usage for expression. The "B" form retains the known proteolytic cleavage sites at the gp120/gp41 junction.

1 GACAATTGGA GGAGCGAGTT ATATAAATAT AAGGTGGTGA AGATTGAGCC

51 CCTGGGGGTG GCCCCAACAA AAGCTAAGAGAAGAGTGGTG CAGAGAGAGA

101 AGAGAGCCGT GGGCATTGGG GCCCTGTTTC TGGGCTTTCT GGGGGCTGCT

151 GGCTCCACAA TGGGCGCCGC TAGCATGACC CTCACCGTGC AAGCTCGCCA

201 GCTGCTGAGT GGCATCGTCC AGCAGCAGAA CAACCTGCTC CGCGCCATCG

251 AAGCCCAGCA GCACCTCCTC CAGCTGACTG TGTGGGGGAT CAAACAGCTT

301 CAGGCCCGGG TGCTGGCCGT CGAGCGCTAT CTGAAAGACC AGCAACTCCT

351 AGGC

### gp32 (opt)

This is a gp32 gene segment from the AvrII site (starting immediately at the end of the RRE) to the end of gp143 comprised of optimal codons for expression.

1 CCTAGGCA TCTGGGGCTG CTCTGGCAAG CTGATCTGCA CCACAGCTGT

51 GCCCTGGAAT GCCTCCTGGT CCAACAAGAG CCTGGAGCAA ATCTGGAACA

101 ACATGACCTG GATGGAGTGG GACAGAGAGA TCAACAACTA CACCTCCCTG

151 ATCCACTCCC TGATTGAGGA GTCCCAGAAC CAGCAGGAGA AGAATGAGCA

201 GGAGCTGCTG GAGCTGGACA AGTGGGCCTC CCTGTGGAAC TGGTTCAACA

251 TCACCAACTG GCTGTGGTAC ATCAAAATCT TCATCATGAT TGTGGGGGGC

301 CTGGTGGGGC TGCGGATTGT CTTTGCTGTG CTGTCCATTG TGAACCGGGT

351 GAGACAGGGC TACTCCCCCT AATAAGCCCG GGCGATATC

### SRV-1 CTE (A)

This is a synthetic gene segment corresponding to a 3'-UTR from the Simian Retrovirus-1 genome. This DNA is placed in the following orientation at the 3'-terminus of HIV genes to increase *rev*-independent expression.

SrfI EcoRV
5'-GCCC GGGC GATATC TA GACCACCTCC CCTGCGAGCT AAGCTGGACA

GCCAATGACG GGTAAGAGAG TGACATTTTT CACTAACCTA AGACAGGAGG

GCCGTCAGAG CTACTGCCTA ATCCAAAGAC GGGTAAAAGT GATAAAAATG

TATCACTCCA ACCTAAGACA GGCGCAGCTT CCGAGGGATT TGTCGTCTGT

TTTATATATA TTTAAAAGGG TGACCTGTCC GGAGCCGTGC TGCCCGGATG

ATGTCTTGG GATATC GCCC GGGC -3'
EcoRV SrfI

### SRV-1 CTE (B)

This synthetic gene segment is identical to SRV-1 CTE (A) shown above except that a single nucleotide mutation was used (indicated by boldface) to eliminate an ATTTA sequence. This sequence has been associated with increased mRNA turnover.

SrfI EcoRV
5'-GCCC GGGC GATATC TA GACCACCTCC CCTGCGAGCT AAGCTGGACA
GCCAATGACG GGTAAGAGAG TGACATTTTT CACTAACCTA AGACAGGAGG
GCCGTCAGAG CTACTGCCTA ATCCAAAGAC GGGTAAAAGT GATAAAAATG
TATCACTCCA ACCTAAGACA GGCGCAGCTT CCGAGGGATT TGTCGTCTGT
TTTATATATA TTAAAAAGGG TGACCTGTCC GGAGCCGTGC TGCCCGGATG
ATGTCTTGG GATATC GCCC GGGC -3'
EcoRV SrfI

### EXAMPLE 12

### In Vitro gp120 Vaccine Expression:

In vitro expression was tested in transfected human rhabdomyosarcoma (RD) cells for these constructs. Quantitation of secreted tPA-gp120 from transfected RD cells showed that V1Jns-tPA-gp 120 vector produced secreted gp120.

### In Vivo gp120 Vaccination:

### See figure 12 (mouse data):

V1Jns-tPA-gp120_{MN} PNV-induced Class II MHC-restricted T lymphocyte gp120 specific antigen reactivities. Balb/c mice which had been vaccinated two times with 200 µg V 1Jns-tPA-gp120_{MN} were sacrificed and their spleens extracted for in vitro determinations of helper T lymphocyte reactivities to recombinant gp120. T cell proliferation assays were performed with PBMC (peripheral blood mononuclear cells) using recombinant gp120_{IIIB} (Repligen, catalogue #RP1016-20) at 5 µg/ml with 4 x 10⁵ cells/ml. Basal levels of 3H-thymidine uptake by these cells were obtained by culturing the cells in media alone, while maximum proliferation was induced using ConA stimulation at 2 µg/ml. ConA-induced reactivities peak at ~3 days and were harvested at that time point with media control samples while antigen-treated samples were harvested at 5 days with an additional media control. Vaccinated mice responses were compared with naive, age-matched syngeneic mice. ConA positive controls gave very high proliferation for both naive and immunized mice as expected. Very strong helper T cell memory responses were obtained by gp120 treatment in vaccinated mice while the naive mice did not respond (the threshold for specific reactivity is an stimulation index (SI) of >3-4; SI is calculated as the ratio of sample cpm/media cpm). SI's of 65 and 14 were obtained for the vaccinated mice which compares with anti-gp120 ELISA titers of 5643 and 11,900, respectively, for these mice. Interestingly, for these two mice the higher responder for antibody gave significantly lower T cell reactivity than the mouse having the lower antibody titer. This experiment demonstrates that the secreted gp120 vector efficiently activates helper T cells in vivo as well as generates strong antiboby responses. In addition, each of these immune responses was determined using antigen which was heterologous compared to that encoded by the inoculation PNV (IIIB vs. MN):

### EXAMPLE 13

### gp160 Vaccines

In addition to secreted gp120 constructs, we have prepared expression constructs for full-length, membrane-bound gp160. The rationales for a gp160 construct, in addition to gp120, are (1) more epitopes are available both for both CTL stimulation as well as neutralizing antibody production including gp41, against which a potent HIV neutralizing monoclonal antibody (2F5, see above) is directed; (2) a more native protein structure may be obtained relative to virus-produced gp160; and, (3) the success of membrane-bound influenza HA constructs for immunogenicity [Ulmer et al., Science 259:1745-1749, 1993; Montgomery, D., et al., DNA and Cell Biol., 12:777-783, 1993]. gp160 retains substantial *REV* dependence even with a heterologous leader peptide sequence so that further constructs were made to increase expression in the absence of *REV.*

### EXAMPLE 14

### Assay For Hiv Cytotoxic T-Lymphocytes:

The methods described in this section illustrate the assay as used for vaccinated mice. An essentially similar assay can be used with primates except that autologous B cell lines must be established for use as target cells for each animal. This can be accomplished for humans using the Epstein-Barr virus and for rhesus monkey using the herpes B virus.

Peripheral blood mononuclear cells (PBMC) are derived from either freshly drawn blood or spleen using Ficoll-Hypaque centrifugation to separate erythrocytes from white blood cells. For mice, lymph nodes may be used as well. Effecter CTLs may be prepared from the PBMC either by in vitro culture in IL-2 (20 U/ml) and concanavalin A (2µg/ml) for 6-12 days or by using specific antigen using an equal number of irradiated antigen presenting cells. Specific antigen can consist of either synthetic peptides (9-15 amino acids usually) that are known eptitopes for CTL recognition for the MHC haplotype of the animals used, or vaccinia virus constructs engineered to express appropriate antigen. Target cells may be either syngeneic or MHC haplotype-matched cell lines which have been treated to present appropriate antigen as described for in vitro stimulation of the CTLs. For Balb/c mice the P 18 peptide (ArgIleHisIleGlyProGlyArgAlaPheTyrThrThrLysAsn, SEQ.ID:51:, for HIV MN strain) can be used at 10 µM concentration to restimulate CTL in vitro using irradiated syngeneic splenocytes and can be used to sensitize target cells during the cytotoxicity assay at 1-10 µM by incubation at 37°C for about two hours prior to the assay. For these H-2^{d} MHC haplotype mice, the murine mastocytoma cell line, P815, provides good target cells. Antigen-sensitized target cells are loaded with Na⁵¹CrO₄, which is released from the interior of the target cells upon killing by CTL, by incubation of targets for 1-2 hours at 37°C (0.2 mCi for ~5 x 10⁶ cells) followed by several washings of the target cells. CTL populations are mixed with target cells at varying ratios of effectors to targets such as 100:1, 50:1, 25:1, etc., pelleted together, and incubated 4-6 hours at 37°C before harvest of the supernatants which are then assayed for release of radioactivity using a gamma counter. Cytotoxicity is calculated as a percentage of total releasable counts from the target cells (obtained using 0.2% Triton X-100 treatment) from which spontaneous release from target cells has been subtracted.

### EXAMPLE 15

### Assay For Hiv Specific Antibodies:

ELISA were designed to detect antibodies generated against HIV using either specific recombinant protein or synthetic peptides as substrate antigens. 96 well microtiter plates were coated at 4°C overnight with recombinant antigen at 2 µg/ml in PBS (phosphate buffered saline) solution using 50 µl/well on a rocking platform. Antigens consisted of either recombinant protein (gp120, *rev*: Repligen Corp.; gp160, gp41: American Bio-Technologies, Inc.) or synthetic peptide (V3 peptide corresponding to virus isolate sequences from IIIB, etc.: American Bio-Technologies, Inc.; gp41 epitope for monoclonal antibody 2F5). Plates were rinsed four times using wash buffer (PBS/0.05% Tween 20) followed by addition of 200µl/well of blocking buffer (1% Carnation milk solution in PBS/0.05% Tween-20) for 1 hr at room temperature with rocking. Pre-sera and immune sera were diluted in blocking buffer at the desired range of dilutions and 100 µl added per well. Plates were incubated for I hr at room temperature with rocking and then washed four times with wash buffer. Secondary antibodies conjugated with horse radish peroxidase, (anti-rhesus Ig, Southern Biotechnology Associates; anti- mouse and anti-rabbit Igs, Jackson Immuno Research) diluted 1:2000 in blocking buffer, were then added to each sample at 100 µl/well and incubated 1 hr at room temperature with rocking. Plates were washed 4 times with wash buffer and then developed by addition of 100 µl/well of an o-phenylenediamine (o-PD, Calbiochem) solution at 1 mg/ml in 100 mM citrate buffer at pH 4.5. Plates were read for absorbance at 450 nm both kinetically (first ten minutes of reaction) and at 10 and 30 minute endpoints (Thermo-max microplate reader, Molecular Devices).

### EXAMPLE 16

### Assay For Hiv Neutralizing Antibodies:

In vitro neutralization of HIV isolates assays using sera derived from vaccinated animals was performed as follows. Test sera and pre-immune sera were heat inactivated at 56°c for 60 min before use. A titrated amount of HIV-1 was added in 1:2 serial dilutions of test sera and incubated 60 min at room temperature before addition to 105 MT-4 human lymphoid cells in 96 well microtiter plates. The virus/cell mixtures were incubated for 7 days at 37°C and assayed for virus-mediated killing of cells by staining cultures with tetrazolium dye. Neutralization of virus is observed by prevention of virus-mediated cell death.

### EXAMPLE 17

### Isolation Of Genes From Clinical Hiv Isolates:

HIV viral genes were cloned from infected PBMC's which had been activated by ConA treatment. The preferred method for obtaining the viral genes was by PCR amplification from infected cellular genome using specific oligomers flanking the desired genes. A second method for obtaining viral genes was by purification of viral RNA from the supernatants of infected cells and preparing cDNA from this material with subsequent PCR. This method was very analogous to that described above for cloning of the murine B7 gene except for the PCR oligomers used and random hexamers used to make cDNA rather than specific priming oligomers.

Genomic DNA was purified from infected cell pellets by lysis in STE solution (10 mM NaCl, 10 mM EDTA, 10 mM Tris-HCl, pH 8.0) to which Proteinase K and SDS were added to 0.1 mg/ml and 0.5% final concentrations, respectively. This mixture was incubated overnight at 56°C and extracted with 0.5 volumes of phenol:chloroform:isoamyl alcohol (25:24:1). The aqueous phase was then precipitated by addition of sodium acetate to 0.3 M final concentration and two volumes of cold ethanol. After pelleting the DNA from solution the DNA was resuspended in 0.1X TE solution (1X TE = 10 mM Tris-HCl, pH 8.0, 1 mM EDTA). At this point SDS was added to 0.1% with 2 U of RNAse A with incubation for 30 minutes at 37°C. This solution was extracted with phenol/chloroform/isoamyl alcohol and then precipitated with ethanol as before. DNA was suspended in 0.1 X TE and quantitated by measuring its ultraviolet absorbance at 260 nm. Samples were stored at -20°C until used for PCR.

PCR was performed using the Perkin-Elmer Cetus kit and procedure using the following sense and antisense oligomers for gp160: 5'-GA AAG AGC AGA AGA CAG TGG CAA TGA -3', and 5'-GGG CTT TGC TAA ATG GGT GGC AAG TGG CCC GGG C ATG TGG-3', respectively. These oligomers add an SrfI site at the 3'-terminus of the resulting DNA fragment. PCR-derived segments are cloned into either the V1Jns or V1R vaccination vectors and V3 regions as well as ligation junction sites confirmed by DNA sequencing.

### EXAMPLE 18

### T Cell Proliferation Assays:

PBMCs are obtained and tested for recall responses to specific antigen as determined by proliferation within the PBMC population. Proliferation is monitored using ³H-thymidine which is added to the cell cultures for the last 18-24 hours of incubation before harvest. Cell harvesters retain isotope-containing DNA on filters if proliferation has occurred while quiescent cells do not incorporate the isotope which is not retained on the filter in free form. For either rodent or primate species 4 X 10⁵ cells are plated in 96 well microtiter plates in a total of 200 µl of complete media (RPMI/10% fetal calf serum). Background proliferation responses are determined using PBMCs and media alone while nonspecific responses are generated by using lectins such as phytohaemagglutin (PHA) or concanavalin A (ConA) at 1- 5 µg/ml concentrations to serve as a positive control. Specific antigen consists of either known peptide epitopes, purified protein, or inactivated virus. Antigen concentrations range from 1- 10 µM for peptides and 1-10 µg/ml for protein. Lectin-induced proliferation peaks at 3-5 days of cell culture incubation while antigen-specific responses peak at 5-7 days. Specific proliferation occurs when radiation counts are obtained which are at least three-fold over the media background and is often given as a ratio to background, or Stimulation Index (SI). HIV gp160 is known to contain several peptides known to cause T cell proliferation of gp160/gp120 immunized or HIV-infected individuals. The most commonly used of these are: T1 (LysGlnIleIleAsnMetTrpGlnGluValGlyLysAlaMetTyrAla,; T2 (HisGluAspIleIleSerLeuTrpAspGlnSerLeuLys); and, TH4 (AspArgValIleGluValValGlnGlyAalTyrArgAlaIleArg). These peptides have been demonstrated to stimulate proliferation of PBMC from antigen-sensitized mice, nonhuman primates, and humans.

### EXAMPLE 19

### Vector V1R Preparation:

In an effort to continue to optimize our basic vaccination vector, we prepared a derivative of V1Jns which was designated as V1R. The purpose for this vector construction was to obtain a minimum-sized vaccine vector, i.e., without unnecessary DNA sequences, which still retained the overall optimized heterologous gene expression characteristics and high plasmid yields that V1J and V1Jns afford. We determined from the literature as well as by experiment that (1) regions within the pUC backbone comprising the E. coli origin of replication could be removed without affecting plasmid yield from bacteria; (2) the 3'-region of the *kan*^{r} gene following the kanamycin open reading frame could be removed if a bacterial terminator was inserted in its stead; and, (3) ~300 bp from the 3'- half of the BGH terminator could be removed without affecting its regulatory function (following the original KpnI restriction enzyme site within the BGH element).

V1R was constructed by using PCR to synthesize three segments of DNA from V1Jns representing the CMVintA promoter/BGH terminator, origin of replication, and kanamycin resistance elements, respectively. Restriction enzymes unique for each segment were added to each segment end using the PCR oligomers: SspI and XhoI for CMVintA/BGH; EcoRV and BamHI for the *kan* ^{r} gene; and, BclI and SalI for the *ori* ^{r}. These enzyme sites were chosen because they allow directional ligation of each of the PCR-derived DNA segments with subsequent loss of each site: EcoRV and SspI leave blunt-ended DNAs which are compatible for ligation while BamHI and BclI leave complementary overhangs as do SalI and XhoI. After obtaining these segments by PCR each segment was digested with the appropriate restriction enzymes indicated above and then ligated together in a single reaction mixture containing all three DNA segments. The 5'-end of the *ori* ^{r} was designed to include the T2 rho independent terminator sequence that is normally found in this region so that it could provide termination information for the kanamycin resistance gene. The ligated product was confirmed by restriction enzyme digestion (>8 enzymes) as well as by DNA sequencing of the ligation junctions. DNA plasmid yields and heterologous expression using viral genes within V1R appear similar to V1Jns. The net reduction in vector size achieved was 1346 bp (V1Jns = 4.86 kb; V1R = 3.52 kb), see figure 11, SEQ.ID:45:.
PCR oligomer sequences used to synthesize V1R (restriction enzyme sites are underlined and identified in brackets following sequence): 
(1) 5'-GGT ACA AAT ATT GG CTA TTG GCC ATT GCA TAC
G-3'
[SspI], SEQ.ID:,
 
(2) 5'-CCA CAT CTC GAG GAA CCG GGT CAA TTC TTC AGC
ACC-3'
[XhoI], SEQ.ID::
(for CMVintA/BGH segment)
 
(3) 5'-GGT ACA GAT ATC GGA AAG CCA CGT TGT GTC TCA
AAA TC-3'[EcoRV], SEQ.ID::
 
(4) 5'-CCA CAT GGA TCC G TAA TGC TCT GCC AGT GTT
ACA ACC-3' [BamHI], SEQ.ID::
(for kanamycin resistance gene segment)
 
(5) 5'-GGT ACA TGA TCA CGT AGA AAA GAT CAA AGG ATC
TTC TTG-3'[BclI], SEQ.ID::,
 
(6) 5'-CCA CAT GTC GAC CC GTA AAA AGG CCG CGT TGC
TGG-3' [SalI], SEQ.ID::
(for E. coli origin of replication)
 Ligation junctions were sequenced for V1R using the following oligomers:

5'-GAG CCA ATA TAA ATG TAC-3', SEQ.ID::
[CMVintA/kan^r junction]

5'-CAA TAG CAG GCA TGC-3', SEQ.ID:: [BGH/ori
junction]

5'-G CAA GCA GCA GAT TAC-3', SEQ.ID:: [ori/kan^r
junction]

### EXAMPLE 20

### Heterologous Expression of HIV Late Gene Products

HIV structural genes such as *env* and *gag* require expression of the HIV regulatory gene, *rev,* in order to efficiently produce full-length proteins. We have found that *rev*-dependent expression of *gag* yielded low levels of protein and that *rev* itself may be toxic to cells. Although we achieved relatively high levels of *rev*-dependent expression of gp160 *in vitro* this vaccine elicited low levels of antibodies to gp160 following in vivo immunization with *rev*/gp160 DNA. This may result from known cytotoxic effects of *rev* as well as increased difficulty in obtaining *rev* function in myotubules containing hundreds of nuclei *(rev* protein needs to be in the same nucleus as a *rev*-dependent transcript in order for *gag* or *env* protein expression to occur). However, it has been possible to obtain *rev*-independent expression using selected modifications of the *env* gene.

### 1. rev-independent expression of env:

In general, our vaccines have utilized primarily HIV (IIIB) *env* and *gag* genes for optimization of expression within our generalized vaccination vector, V1Jns, which is comprised of a CMV immediate-early (IE) promoter, a BGH-derived polyadenylation and transcriptional termination sequence, and a pUC backbone. Varying efficiencies, depending upon how large a gene segment is used (e.g., gp 120 vs. gp160), of *rev*-independent expression may be achieved for *env* by replacing its native secretory leader peptide with that from the tissue-specific plasminogen activator (tPA) gene and expressing the resulting chimeric gene behind the CMVIE promoter with the CMV intron A. tPA-gp120 is an example of a secreted gp 120 vector constructed in this fashion which functions well enough to elicit anti-gp120 immune responses in vaccinated mice and monkeys.

Because of reports that membrane-anchored proteins may induce much more substantial (and perhaps more specific for HIV neutralization) antibody responses compared to secreted proteins as well as to gain additional epitopes, we prepared V 1Jns-tPA-gp160 and V1Jns-*rev*/gp160. The tPA-gp160 vector produced detectable quantities of gp 160 and gp 120, without the addition of *rev,* as shown by immunoblot analysis of transfected cells, although levels of expression were much lower than that obtained for *rev*/gp160, a *rev*-dependent gp 160-expressing plasmid. This is probably because inhibitory regions, which confer *rev* dependence upon the gp160 transcript, occur at multiple sites within gp160 including at the COOH-terminus of gp41. A vector was prepared for a COOH-terminally truncated form of tPA-gp160 (tPA-gp143) which was designed to increase the overall expression levels of *env* by elimination of these inhibitory sequences. The gp 143 vector also eliminates intracellular gp41 regions containing peptide motifs (such as Leu-Leu) known to cause diversion of membrane proteins to the lysosomes rather than the cell surface. Thus, gp143 may be expected to have increased levels of expression of the *env* protein (by decreasing *rev*-dependence) and greater efficiency of transport of protein to the cell surface compared to full-length gp160 where these proteins may be better able to elicit anti-gp160 antibodies following DNA vaccination. tPA-gp143 was further modified by extensive silent mutagenesis of the *rev* response element (RRE) sequence (350 bp) to eliminate additional inhibitory sequences for expression. This construct, gp143/mutRRE, was prepared in two forms: either eliminating (form A) or retaining (form B) proteolytic cleavage sites for gp120/41. Both forms were prepared because of literature reports that vaccination of mice using uncleavable gp160 expressed in vaccinia elicited much higher levels of antibodies to gp160 than did cleavable forms.

A quantitative ELISA for gp160/gp120 expression in cell transfectants was developed to determine the relative expression capabilities for these vectors. In vitro transfection of 293 cells followed by quantification of cell-associated vs. secreted/released gp120 yielded the following results: (1) tPA-gp160 expressed 5-10X less gp120 than *rev*/gp160 with similar proportions retained intracellularly vs. released from the cell surface; (2) tPA-gp143 gave 3-6X greater secretion of gp120 than *rev*/gp160 with only low levels of cell-associated gp143, confirming that the cytoplasmic tail of gp160 causes intracellular retention of gp160 which can be overcome by partial deletion of this sequence; and, (3) tPA-gp143/mutRRE A and B gave ~10X greater expression levels of protein than did parental tPA-gp143 while elimination of proteolytic processing was confirmed for form A.

Thus, our strategy to increase *rev*-independent expression has yielded stepwise increases in overall expression levels as well as redirecting membrane-anchored gp143 to the cell surface away from lysosomes. It is important to note that this is a generic construct into which it should be possible to insert gp120 sequences derived from various primary viral isolates within a vector cassette containing these modifications which reside either at the NH2-terminus (tPA leader) or COOH-terminus (gp41), where few antigenic differences exist between different viral strains.

### 2. Expression of gp120 derived from a clinical isolate:

To apply these expression strategies to viruses that are relevant for vaccine purposes and confirm the generality of our approaches, we also prepared a tPA-gp120 vector derived from a primary HIV isolate (containing the North American concensus V3 peptide loop; macrophage-tropic and nonsyncytia-inducing phenotypes). This vector gave high expression/secretion of gp120 with transfected 293 cells and elicited anti-gp120 antibodies in mice thus demonstrating that it was cloned in a functional form. Primary isolate gp160 genes will also be used for expression in the same way as for gp160 derived from laboratory strains.

### B. Immune Responses to HIV-1 env Polynucleotide Vaccines

Effect of vaccination route on immune responses in mice: While efforts to improve expression of gp160 are ongoing, we have utilized the tPA-gp 120 DNA construct to assess immune responses and ways to augment them. Intramuscular (i.m.) and intradermal (i.d.) vaccination routes were compared for this vector at 100, 10, and 1 µg doses in mice. Vaccination by either route elicited antibody responses (GMTs = 10³-10⁴) in all recipients following 2-3 vaccinations at all three dosage levels. Each route elicited similar anti-gp120 antibody titers with clear dose-dependent responses. However, we observed greater variability of responses for i.d. vaccination, particularly at the lower doses following the initial inoculation. Moreover, helper T-cell responses, as determined by antigen-specific in vitro proliferation and cytokine secretion, were higher following i.m. vaccination than i.d. We concluded that i.d. vaccination did not offer any advantages compared to i.m. for this vaccine.

### 2. gp120 DNA vaccine-mediated helper T cell immunity in mice:

gp120 DNA vaccination produced potent helper T-cell responses in all lymphatic compartments tested (spleen, blood, inguinal, mesenteric, and iliac nodes) with T_{H}1-like cytokine secretion profiles (i.e., g-interferon and IL-2 production with little or no IL-4). These cytokines generally promote strong cellular immunity and have been associated with maintenance of a disease-free state for HIV-seropositive patients. Lymph nodes have been shown to be primary sites for HIV replication, harboring large reservoirs of virus even when virus cannot be readily detected in the blood. A vaccine which can elicit anti-HIV immune responses at a variety of lymph sites, such as we have shown with our DNA vaccine, may help prevent successful colonization of the lymphatics following initial infection.

### 3. env DNA vaccine-mediated antibody responses:

African green (AGM) and Rhesus (RHM) monkeys which received gp120 DNA vaccines showed low levels of neutralizing antibodies following 2-3 vaccinations, which could not be increased by additional vaccination. These results, as well as increasing awareness within the HIV vaccine field that oligomeric gp160 is probably a more relevant target antigen for eliciting neutralizing antibodies than gp120 monomers, have led us to focus upon obtaining effective expression of gp160-based vectors (see above). Mice and AGM were also vaccinated with the primary isolate derived tPA-gp120 vaccine. These animals exhibited anti-V3 peptide (using homologous sequence) reciprocal endpoint antibody titers ranging 500-5000, demonstrating that this vaccine design is functional for clinically relevant viral isolates.

The gp160-based vaccines, *rev*-gp160 and tPA-gp 160, failed to consistently elicit antibody responses in mice and nonhuman primates or yielded low antibody titers. Our initial results with the tPA-gp143 plasmid yielded geometric mean titers (GMT) > 10³ in mice and AGM following two vaccinations. These data indicate that we have signficantly improved the immunogenicity of gp160-like vaccines by increasing expression levels and more efficient intracellular trafficking of *env* to the cell surface. This construct, as well as the tPA-gp143/mutRRE A and B vectors, will continue to be characterized for antibody responses, especially for virus neutralization.

### 4. env DNA vaccine-mediated CTL responses in monkeys:

We continued to characterize CTL responses of RHM that had been vaccinated with gp120 and gp160/IRES/*rev* DNA. All four monkeys that received this vaccine showed significant MHC Class I-restricted CTL activities (20-35% specific killing at an effector/target = 20) following two vaccinations. Following a fourth vaccination these activities increased to 50-60% killing under similar test conditions, indicating that additional vaccination boosted responses significantly. The CTL activities have persisted for at least seven months subsequent to the final vaccination at about 50% of their peak levels indicating that long-term memory had been established.

## Claims

1. A synthetic polynucleotide comprising a DNA sequence encoding HIV *env* protein, the DNA sequence comprising codons optimized for expression in a mammalian host, wherein the polynucleotide is selected from:
V1Jns-tPA-gp140/mutRRE-A/SRV-1 3'-UTR;
V1Jns-tPA-gp140/mutRRE-B/SRV-1 3'-UTR;
V 1Jns-tPA-gp140/opt30-A;
V1Jns-tPA-gp140/opt30-B;
V1Jns-tPA-gp140/opt all-A;
V1Jns-tPA-gp140/opt all-B (non-IIIB strains);
V1Jns-tPA-gp140/opt all-A (non-IIIB strains);
V1Jns-tPA-gp140/opt all-B;
V1Jns-tPA-gp143/mutRRE-A;
V1Jns-tPA-gp143/mutRRE-B;
V1Jns-tPA-gp143/opt32-A;
V1Jns-tPA-gp143/opt32-B;
V1Jns-tPA-gp143/SRV-1 3'-UTR;
V1Jns-tPA-gp143/opt C1/opt32A;
V1Jns-tPA-gp143/opt C1/opt32B;
V1Jns-tPA-gp143/opt all-A;
V1Jns-tPA-gp143/opt all-B;
V1Jns-tPA-gp143/opt all-A;
V1Jns-tPA-gp143/opt all-B;
V1Jns-tPA-gp143/opt32-A/glyB;
V1Jns-tPA-gp143/opt32-B/glyB;
V1Jns-tPA-gp143/opt CI/opt32-A/glyB;
V1Jns-tPA-gp143/opt C1/opt32-B/glyB;
V1Jns-tPA-gp143/opt all-A/glyB;
V1Jns-tPA-gp143/opt all-B/glyB:
V1Jns-tPA-gp143/opt all-A/glyB;
V1Jns-tPA-gp143/opt all-B/glyB; (as described in examples 1-11 herein); and combinations thereof.

2. The use of the polynucleotide of Claim 1 for the manufacture of a medicament for prevention or treatment of HIV infection in a vertebrate.

3. A vaccine against HIV infection which comprises the polynucleotide of Claim 1 and a pharmaceutically acceptable carrier.

4. The use according to claim 2 wherein said medicament induces an antigen presenting cell to stimulate cytotoxic and helper T-cell proliferation and effector functions including lymphokine secretion specific to HIV antigens.

## Patentansprüche

1. Synthetisches Polynukleotid, umfassend eine DNA-Sequenz, die für HIV-*env*-Protein kodiert, wobei die DNA-Sequenz Codons umfasst, welche für die Expression in einem Säugerwirt optimiert sind, wobei das Polynukleotid ausgewählt ist aus:
V1Jns-tPA-gp140/mutRRE-A/SRV-1-3'-UTR;
V1Jns-tPA-gp140/mutRRE-B/SRV-1-3'-UTR;
V1Jns-tPA-gp140/opt30-A;
V1Jns-tPA-gp140/opt30-B;
V1Jns-tPA-gp140/opt all-A;
V1Jns-tPA-gp140/opt all-B (Nicht-IIIB-Stämme);
V1Jns-tPA-gp140/opt all-A (Nicht-IIIB-Stämme);
V1Jns-tPA-gp140/opt all-B;
V1Jns-tPA-gp143/mutRRE-A;
V1 Jns-tPA-gp143/mutRRE-B;
V1Jns-tPA-gp143/opt32-A;
V1Jns-tPA-gp143/opt32-B;
V1Jns-tPA-gp143/SRV-1-3'-UTR;
V1Jns-tPA-gp143/opt C1/opt32A;
V1Jns-tPA-gp143/opt C1/opt32B;
V1Jns-tPA-gp143/opt all-A;
V1Jns-tPA-gp143/opt all-B;
V1Jns-tPA-gp143/opt all-A;
V 1Jns-tPA-gp143/opt all-B;
V 1Jns-tPA-gp143/opt32-A/glyB;
V1Jns-tPA-gp143/opt32-B/glyB;
V1Jns-tPA-gp143/opt C1/opt32-A/glyB;
V1Jns-tPA-gp143/opt C1/opt32-B/glyB;
V1Jns-tPA-gp143/opt all-A/glyB;
V1Jns-tPA-gp143/opt all-B/glyB;
V1Jns-tPA-gp143/opt all-A/glyB;
V1Jns-tPA-gp143/opt all-B/glyB; (wie hier in den Beispielen 1-11 beschrieben); und Kombinationen davon.

2. Verwendung des Polynukleotids nach Anspruch 1 zur Herstellung eines Medikaments zur Verhütung oder Behandlung einer HIV-Infektion bei einem Vertebraten.

3. Vakzin gegen eine HIV-Infektion, welches das Polynukleotid nach Anspruch 1 und einen pharmazeutisch annehmbaren Träger umfasst.

4. Verwendung nach Anspruch 2, wobei das Medikament eine antigen-präsentierende Zelle zur Stimulation der Proliferation von zytotoxischen und Helfer-T-Zellen und zur Stimulation von Effektor-Funktionen, einschließlich für HIV-Antigene spezifischer Lymphokin-Sekretion, veranlasst.

## Revendications

1. Polynucléotide synthétique comprenant une séquence d'ADN codant pour la protéine *env* du VIH, la séquence d'ADN comprenant des codons optimisés pour l'expression dans un hôte mammalien, dans lequel le polynucléotide est choisi parmi :
V1Jns-tPA-gp140/mutRRE-A/SRV-1 3'-UTR,
V1Jns-tPA-gp140/mutRRE-B/SRV-1 3'-UTR,
V1Jns-tPA-gp140/opt30-A,
V1Jns-tPA-gp140/opt30-B,
V1Jns-tPA-gp140/opt all-A,
V1Jns-tPA-gp140/opt all-B (souches non-IIIB),
V1Jns-tPA-gp140/opt all-A (souches non-IIIB),
V1Jns-tPA-gp140/opt all-B,
V1Jns-tPA-gp143/mutRRE-A,
V1Jns-tPA-gp143/mutRRE-B,
V1Jns-tPA-gp143/opt32-A,
V1Jns-tPA-gp143/opt32-B,
V1Jns-tPA-gp143/SRV-1 3'-UTR,
V1Jns-tPA-gp143/opt C1/opt32A,
V1Jns-tPA-gp143/opt C1/opt32B,
V1Jns-tPA-gp143/opt all-A,
V1Jns-tPA-gp143/opt all-B,
V1Jns-tPA-gp143/opt all-A,
V1Jns-tPA-gp143/opt all-B,
V1Jns-tPA-gp143/opt32-A/glyB,
V1Jns-tPA-gp143/opt32-B/glyB,
V1Jns-tPA-gp143/opt C1/opt32-A/glyB,
V1Jns-tPA-gp143/opt C1/opt32-B/glyB,
V1Jns-tPA-gp143/opt all-A/glyB,
V1Jns-tPA-gp143/opt all-B/glyB,
V1Jns-tPA-gp143/opt all-A/glyB,
V1Jns-tPA-gp143/opt all-B/glyB, (tels que décrits dans les présents exemples 1-11) et des associations de ceux-ci.

2. Utilisation du polynucléotide selon la revendication 1, pour la fabrication d'un médicament destiné à la prévention ou au traitement d'une infection à VIH chez un vertébré.

3. Vaccin contre une infection à VIH, comprenant le polynucléotide selon la revendication 1 et un véhicule pharmaceutiquement acceptable.

4. Utilisation selon la revendication 2, dans laquelle ledit médicament induit une cellule présentant un antigène à stimuler la prolifération et les fonctions effectrices, incluant la sécrétion de lymphokines spécifiques contre des antigènes du VIH, des lymphocytes T cytotoxiques et auxiliaires.
